(19) Europäisches Patentamt
European Patent Office
Office européen des brevets

(11) **EP 4 332 118 A1**

(12) **EUROPEAN PATENT APPLICATION**
published in accordance with Art. 153(4) EPC

(43) Date of publication:
**06.03.2024 Bulletin 2024/10**

(21) Application number: **22796130.7**

(22) Date of filing: **27.04.2022**

(51) International Patent Classification (IPC):
**C07K 16/28** (2006.01)  **C12N 5/0783** (2010.01)
**A61K 35/17** (2015.01)  **A61P 35/00** (2006.01)

(52) Cooperative Patent Classification (CPC):
**A61K 35/17; A61K 39/00; A61P 35/00;
C07K 14/705; C07K 16/28; C12N 5/06**

(86) International application number:
**PCT/KR2022/006009**

(87) International publication number:
**WO 2022/231298 (03.11.2022 Gazette 2022/44)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **29.04.2021 KR 20210055886**

(71) Applicant: **Korea Research Institute of Bioscience and
Biotechnology
Daejeon 34141 (KR)**

(72) Inventors:
• **KIM, Tae-Don**
  **Daejeon 34141 (KR)**
• **CHO, Seona**
  **Daejeon 34141 (KR)**
• **LEE, Sooyun**
  **Daejeon 34141 (KR)**

(74) Representative: **Dehns
St. Bride's House
10 Salisbury Square
London EC4Y 8JD (GB)**

Remarks:
The complete document including Reference
Table(s) and the Sequence Listing(s) can be
downloaded from the EPO website

(54) **NOVEL ANTI-CD5 CHIMERIC ANTIGEN RECEPTOR AND IMMUNE CELL EXPRESSING SAME**

(57)    The present invention relates to a CD5 antibody and a chimeric antigen receptor (CAR) including the same. The chimeric antigen receptor of the present invention may be expressed in an immune cell to be utilized for an immuno-oncology therapy having reduced toxicity against a normal T cell.

FIG. 1

**Description**

## TECHNICAL FIELD

The present invention relates to a novel antibody or an antigen-binding fragment thereof, which specifically binds to CD5, a chimeric antigen receptor including the antigen-binding fragment of the antibody, and immune cells expressing the chimeric antigen receptors.

## BACKGROUND ART

The methods for treating cancer have been subjected to the process of steady development and change, and although methods such as a surgical operation, chemotherapy, and radiotherapy have been continuously used so far, these existing methods for treating cancer are mostly effective only in the initial state in which the cancer has not metastasized, and there is a limitation in that, in a state in which the metastasis has already progressed, for example, even if a surgical operation is performed, there is high probability of recurrence in the future. Accordingly, recently, studies on a method for using an immune response to treat cancer have been continued.

Among them, interest in cell therapy methods in which immune cells are used to enhance or genetically modify the immune cells to inject the cells back into patients has increased, and for example, technologies of tumor infiltrating lymphocytes (TIL), chimeric antigen receptors (CAR), and T-cell receptors (TCR) have been studied. In particular, in the case of CAR-T cells that are T cells into which the chimeric antigen receptor (CAR), which is an artificial receptor designed to deliver antigen specificity, is introduced, the studies have been actively conducted since CAR-T cells targeting CD19 were approved by FDA as anticancer agents in 2017, but most of them have an effect only *in vitro* and do not actually show a significant therapeutic effect *in vivo.*

Meanwhile, the CAR-T cells are effective for tumors, but in some cases, there are adverse effects of nonspecifically attacking healthy tissue. The CD5 is a type of differentiation cluster expressed in T cells and B cells, and is mainly found in bone marrow or lymph tissues, and is mainly used as a marker for T cells because the CD5 is overexpressed in T cells rather than B cells. In particular, since the CD5 is expressed in normal T cells as well as in most T-cell neoplasm, there was a limitation of self-cross reaction in that anti-CD5 CAR-T cells attacked not only the T-cell neoplasm but also normal T cells, or even the injected anti-CD5 CAR-T cells themselves (Document 1). Therefore, there is a limitation in that normal T cells and anti-CD5 CAR-T cells are reduced, and eventually even the anticancer effect deteriorates.

Accordingly, there is a need for research and development of immune cells capable of supplementing the above limitations of anti-CD5 CAR-T cells while maintaining the anti-cancer activity against cancer cells expressing CD5.

[PRIOR ART DOCUMENTS]

[PATENT DOCUMENT]

(Patent Document 1) Korean Patent Laid-open Publication No. 2018-0002604
(Patent Document 2) Korean Patent No. 2,122,546

## DISCLOSURE OF THE INVENTION

## TECHNICAL PROBLEM

The purpose of the present invention is to provide an antibody or an antigen-binding fragment thereof which can specifically bind to CD5.

In addition, the purpose of the present invention is to provide a novel chimeric antigen receptor which can amplify cytotoxicity or cytolytic activity against cancer cells when expressed in an immune cell.

In addition, the purpose of the present invention is to provide a polynucleotide and an expression vector for expressing the chimeric antigen receptor.

In addition, the purpose of the present invention is to provide an immune cell having an excellent effect of treating cancer by expressing the chimeric antigen receptor on the surface thereof.

In addition, the purpose of the present invention is to provide a pharmaceutical composition for treating cancer using the immune cells.

## TECHNICAL SOLUTION

To achieve the above purposes, an aspect of the present invention, there is provided an antibody or an antigen-binding fragment thereof, specifically binding to CD5, including: a heavy chain variable region including i) a heavy chain CDR1 having any one amino acid sequence selected from the group consisting of SEQ ID NO: 2, SEQ ID NO: 10, SEQ ID NO: 18, and SEQ ID NO: 26, ii) a heavy chain CDR2 having any one amino acid sequence selected from the group consisting of SEQ ID NO: 3, SEQ ID NO: 11, SEQ ID NO: 19, and SEQ ID NO: 27, and iii) a heavy chain CDR3 having any one amino acid sequence selected from the group consisting of SEQ ID NO: 4, SEQ ID NO: 12, SEQ ID NO: 20, and SEQ ID NO: 28; and a light chain variable region including iv) a light chain CDR1 having any one amino acid sequence selected from the group consisting of SEQ ID NO: 6, SEQ ID NO: 14, SEQ ID NO: 22, and SEQ ID NO: 30, v) a light chain CDR2 having any one amino acid sequence selected from the group consisting of SEQ ID NO: 7, SEQ ID NO: 15, SEQ ID NO: 23, and SEQ ID NO: 31, and vi) a light chain CDR3 having any one amino acid sequence selected from the group consisting of SEQ ID NO: 8, SEQ ID NO: 16, SEQ ID NO: 24, and SEQ ID NO: 32.

The heavy chain variable region may be any one selected from the group consisting of SEQ ID NO: 5, SEQ ID NO: 13, SEQ ID NO: 21, and SEQ ID NO: 29.

The light chain variable region may be any one selected from the group consisting of SEQ ID NO: 9, SEQ ID NO: 17, SEQ ID NO: 25, and SEQ ID NO: 33.

Another aspect of the present invention, there is provided a chimeric antigen receptor (CAR) including an extracellular binding domain including an antigen-binding site specifically binding to CD5, a transmembrane domain, and an intracellular signaling domain, wherein the antigen-binding site specifically binding to the CD5 is a single chain variable fragment (scFv) of an anti-CD5 antibody including: a heavy chain variable region including i) a heavy chain CDR1 having any one amino acid sequence selected from the group consisting of SEQ ID NO: 2, SEQ ID NO: 10, SEQ ID NO: 18, and SEQ ID NO: 26, ii) a heavy chain CDR2 having any one amino acid sequence selected from the group consisting of SEQ ID NO: 3, SEQ ID NO: 11, SEQ ID NO: 19, and SEQ ID NO: 27, and iii) a heavy chain CDR3 having any one amino acid sequence selected from the group consisting of SEQ ID NO: 4, SEQ ID NO: 12, SEQ ID NO: 20, and SEQ ID NO: 28; and a light chain variable region including iv) a light chain CDR1 having any one amino acid sequence selected from the group consisting of SEQ ID NO: 6, SEQ ID NO: 14, SEQ ID NO: 22, and SEQ ID NO: 30, v) a light chain CDR2 having any one amino acid sequence selected from the group consisting of SEQ ID NO: 7, SEQ ID NO: 15, SEQ ID NO: 23, and SEQ ID NO: 31, and vi) a light chain CDR3 having any one amino acid sequence selected from the group consisting of SEQ ID NO: 8, SEQ ID NO: 16, SEQ ID NO: 24, and SEQ ID NO: 32.

Yet another aspect of the present invention, there is provided a polynucleotide including a base sequence encoding the chimeric antigen receptor, and an expression vector including the polynucleotide.

Still another aspect of the present invention, there is provided an immune cell that expresses the chimeric antigen receptor on the surface thereof.

The immune cell may be a natural killer cell, a T cell, a natural killer T cell, a cytokine-induced killer cell, a macrophage, or a dendritic cell. Still yet another aspect of the present invention provides a pharmaceutical composition for preventing or treating cancer, the pharmaceutical composition including the immune cell as an active ingredient.

The cancer may be blood cancer including leukemia or lymphoma, and the cancer may be thymic carcinoma, non-Hodgkin lymphoma, diffuse large cell lymphoma, small lymphocytic lymphoma, T-cell neoplasm, peripheral T-cell lymphoma, mantle cell lymphoma, T-cell acute lymphoblastic leukemia, or chronic lymphocytic leukemia.

## ADVANTAGEOUS EFFECTS

The antibody, the antigen-binding fragment thereof, and the chimeric antigen receptor using the same of the present invention can specifically bind to CD5 expressed in cancer cells, and thus, a signaling pathway is triggered in the immune cells in which the chimeric antigen receptor is expressed, thereby significantly improving the cytotoxicity or cytolytic activity of the immune cell and promoting the secretion of cytokines. In addition, accordingly, there is an effect of increasing the degranulation of the cancer cells co-cultured with the immune cells, as well as the immune cells expressing the chimeric antigen receptors have a reduced reactivity with respect to normal cells expressing CD5.

Therefore, the antibody or the chimeric antigen receptor including the same, and the immune cell expressing the same of the present invention can be used for treating cancer.

However, the effects of the present invention are not limited to the above-mentioned effects and other effects not mentioned will be clearly understood from the following description by a person skilled in the art.

## BRIEF DESCRIPTION OF THE DRAWINGS

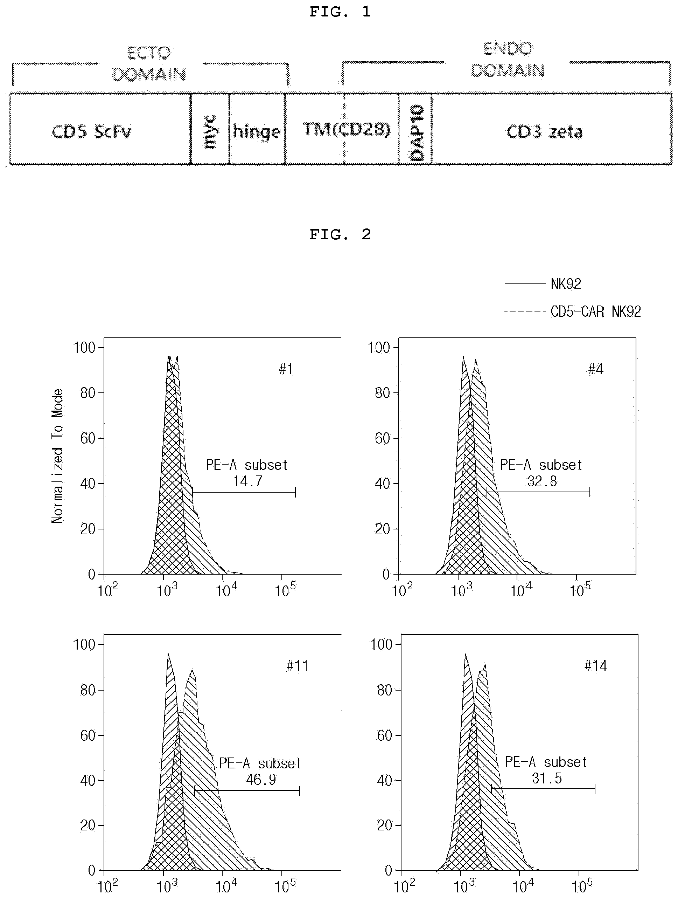

FIG. 1 is a schematic diagram showing the structure of a chimeric antigen receptor (CD5-CAR) of the present invention including, as an extracellular domain, a single chain variable fragment (ScFv) specifically binding to CD5.

FIG. 2 shows the results confirming whether the chimeric antigen receptors (CD5#1-CAR, CD5#4-CAR, CD5#11-CAR, and CD5#14-CAR) of the present invention are expressed in natural killer cells (CD5#1-CAR-NK cells, CD5#4-CAR-NK cells, CD5#11-CAR-NK cells, and CD5#14-CAR-NK cells) in which genes of four chimeric antigen receptors of the present invention have been introduced and expressed.

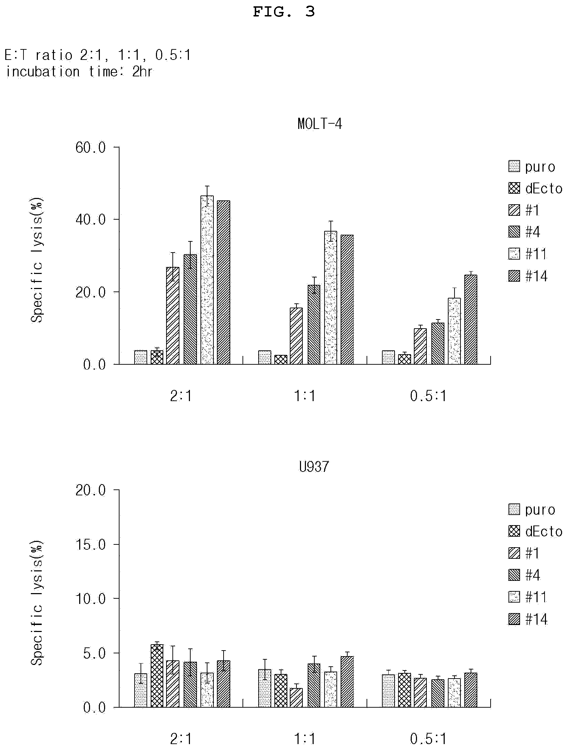

FIG. 3 shows the results obtained by measuring and comparing the cytotoxicity (cytolytic activity) of the natural killer cells (CD5#1-CAR-NK cells, CD5#4-CAR-NK cells, CD5#11-CAR-NK cells, and CD5#14-CAR-NK cells), in which genes of four chimeric antigen receptors of the present invention have been introduced and expressed, against MOLT4 cells expressing CD5 and U937 cells not expressing CD5.

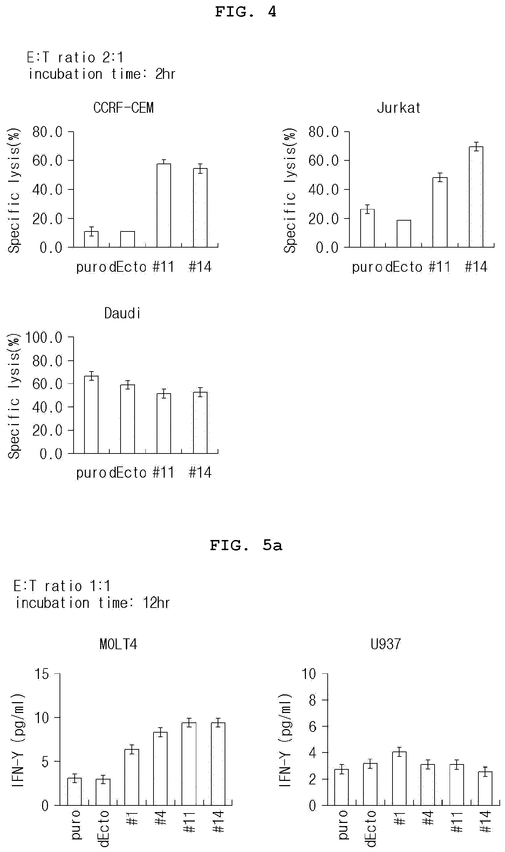

FIG. 4 shows the results obtained by measuring and comparing the cytotoxicity (cytolytic activity) of the natural killer cells (CD5#11-CAR-NK cells and CD5#14-CAR-NK cells), in which genes of the chimeric antigen receptors, CD5#11-CAR and CD5#14-CAR of the present invention have been introduced and expressed, against CCRF-CEM cells expressing CD5, Jurkat cells expressing CD5, and Daudi cells not expressing CD5.

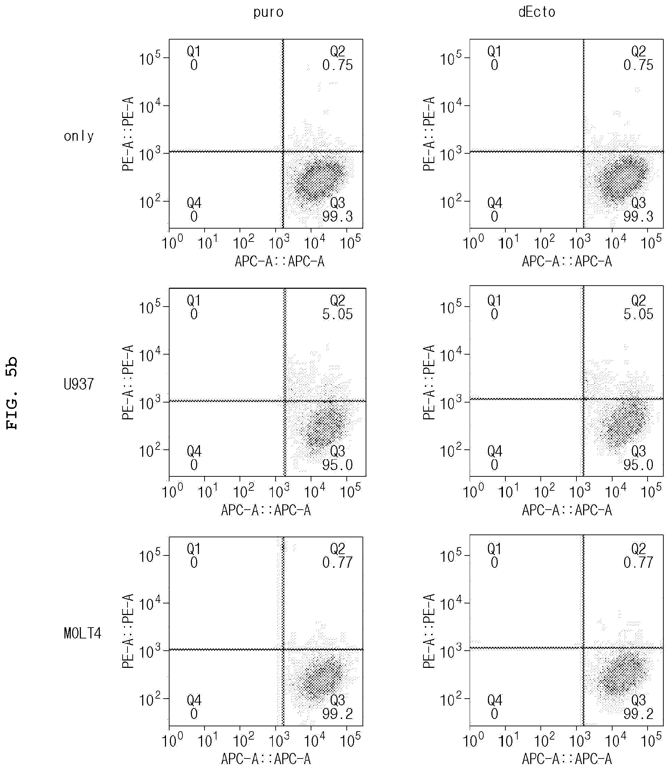

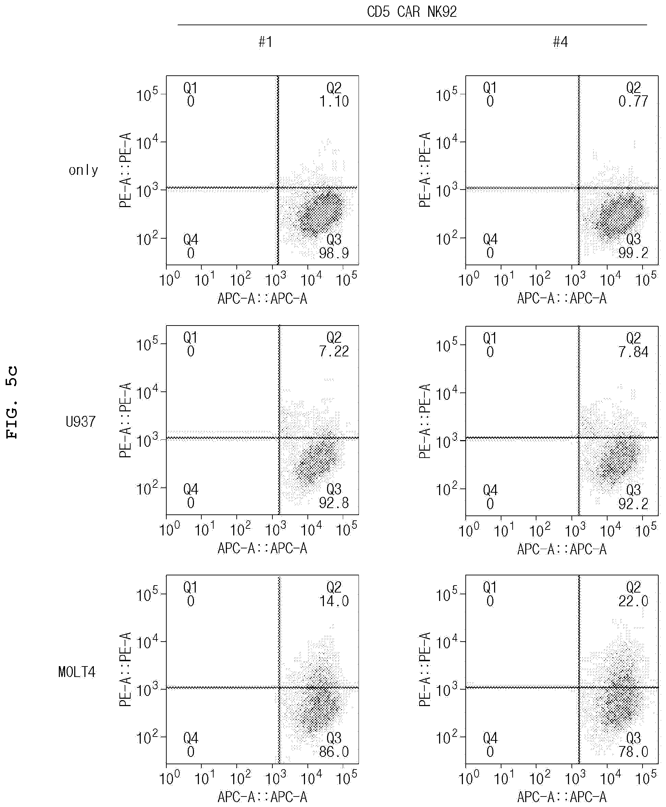

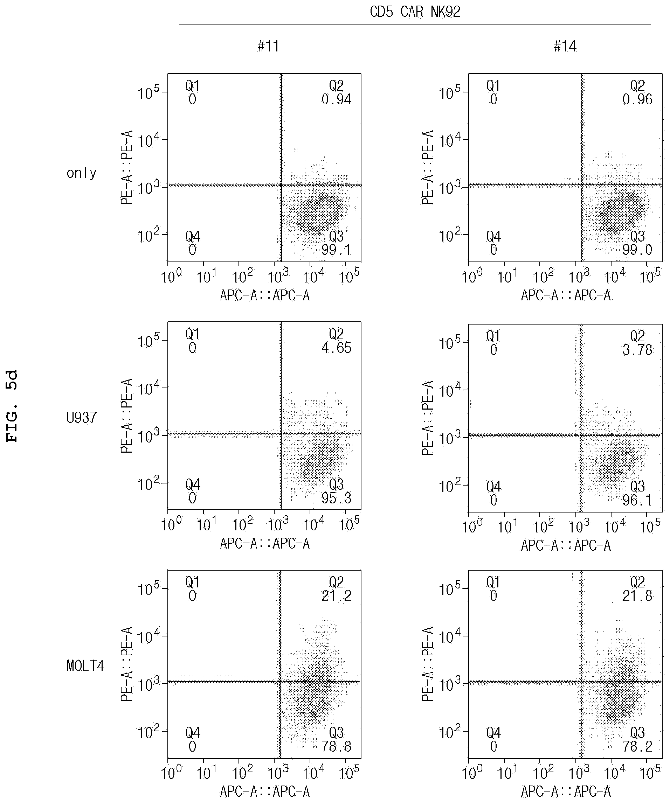

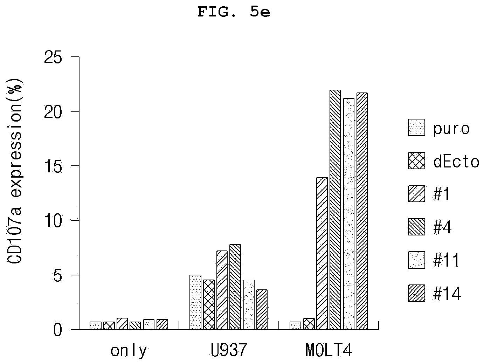

FIGS. 5a, and 5b to 5e show results obtained by respectively measuring and comparing amounts of cytokines (IFN-$\gamma$) secreted from the NK cells (FIG. 5A) and expression levels of Cd107$\alpha$, which are degranulation indicators (FIGS. 5B to 5E), when NK cells (CD5#1-CAR-NK cells, CD5#4-CAR-NK cells, CD5#11-CAR-NK cells, and CD5#14-CAR-NK cells), in which genes of four chimeric antigen receptors of the present invention have been introduced and expressed, are co-cultured with MOLT4 cells expressing CD5 or U937 cells not expressing CD5.

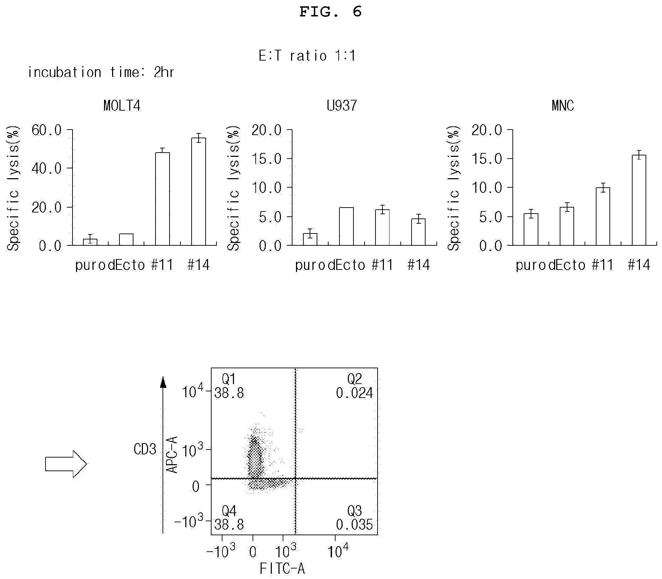

FIG. 6 shows the results obtained by measuring and comparing cytotoxicity (cytolytic activity) of natural killer cells (CD5#11-CAR-NK cells and CD5#14-CAR-NK cells), in which genes of chimeric antigen receptors, CD5#11-CAR and CD5#14-CAR of the present invention have been introduced and expressed, against mononuclear cells (MNCs) including normal cells expressing CD5.

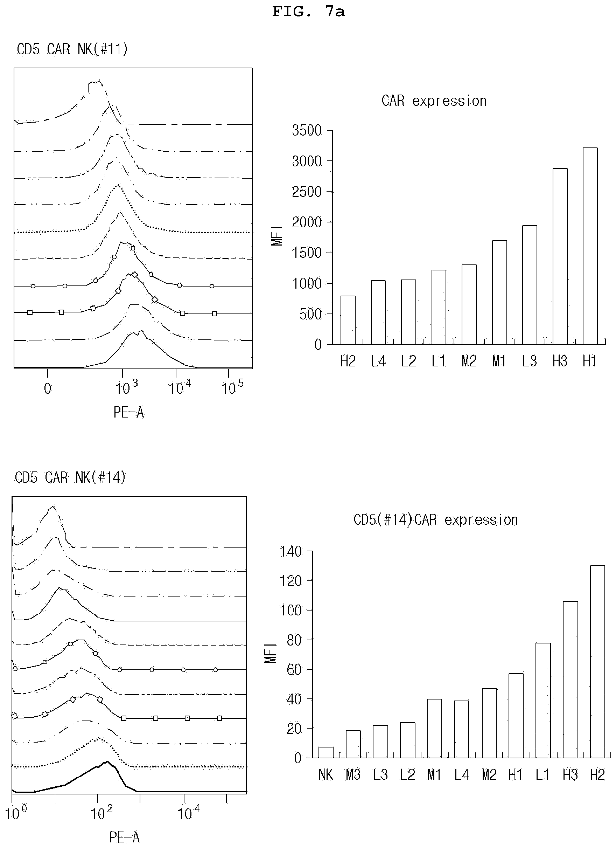

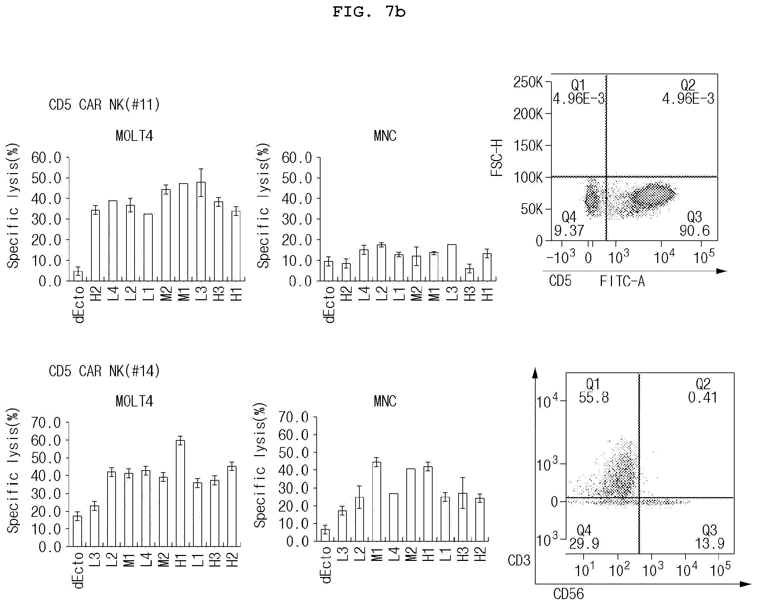

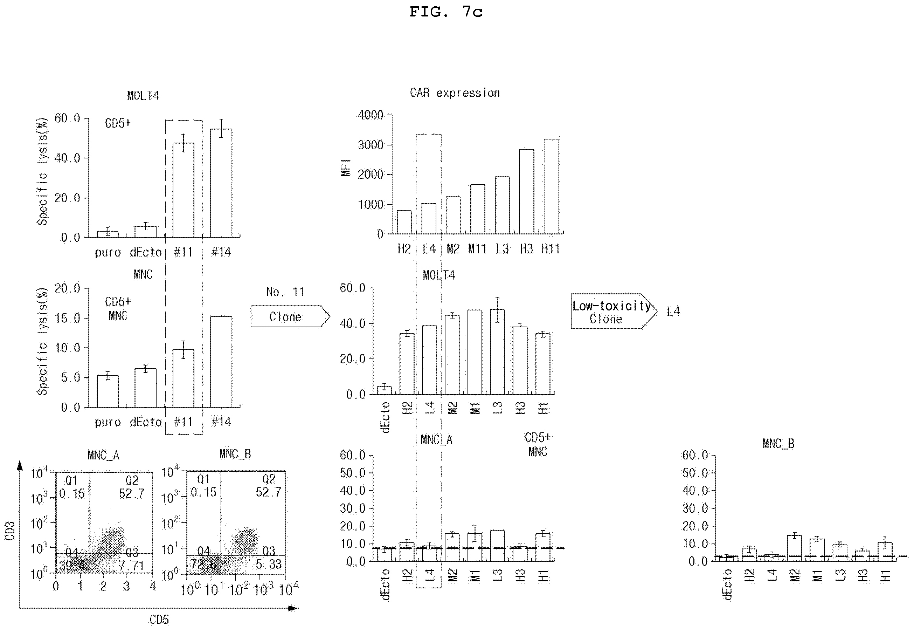

FIG. 7a shows the results confirming the expression level of CD5-CAR of single cell lines obtained by separating and proliferating, into single cells, natural killer cells (CD5#11-CAR-NK cells and CD5#14-CAR-NK cells), in which genes of the chimeric antigen receptors, CD5#11-CAR and CD5#14-CAR of the present invention have been introduced and expressed, FIG. 7b shows the results obtained by measuring and comparing cytotoxicity (cytolytic activity) of each of the established single cell lines against MOLT4 cells and MNC, and FIG. 7c shows the process of selecting a leading cell line among the established single cell lines.

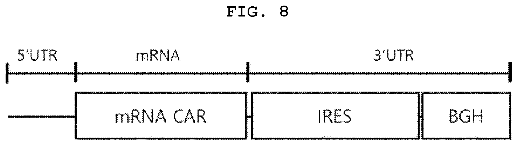

FIG. 8 is a schematic diagram showing the configuration of an mRNA structure designed to temporarily express the chimeric antigen receptor (CD5-CAR) of the present invention.

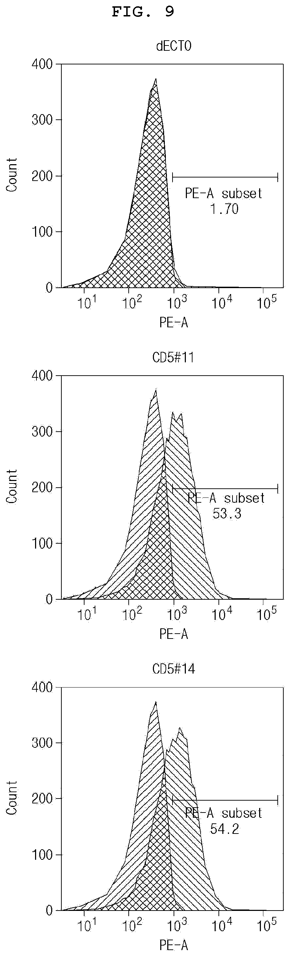

FIG. 9 shows the results confirming whether chimeric antigen receptors (CD5#11-CAR and CD5#14-CAR) of the present invention are expressed in primary natural killer cells in which genes of chimeric antigen receptors, CD5#11-CAR and CD5#14-CAR, of the present invention have been introduced and expressed.

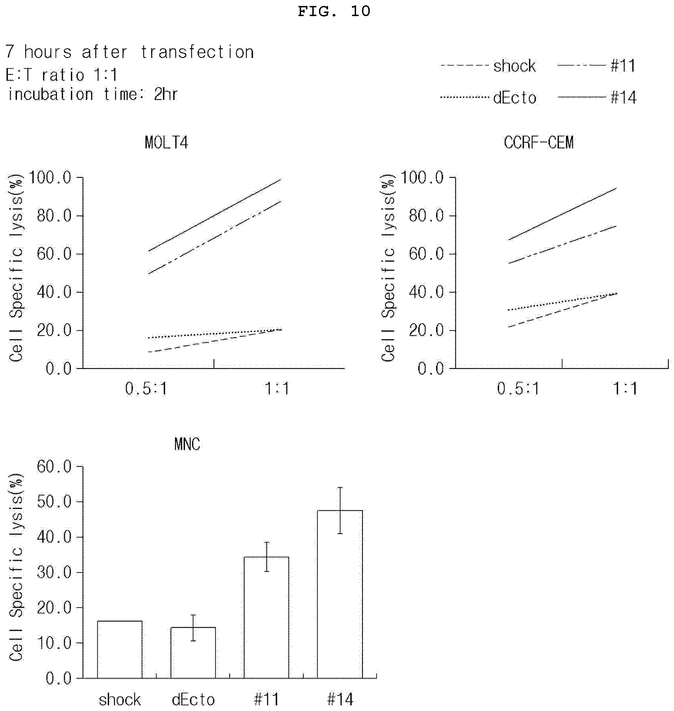

FIG. 10 shows the results obtained by measuring and comparing the cytotoxicity (cytolytic activity) of primary natural killer cells, in which genes of the chimeric antigen receptors, CD5#11-CAR and CD5#14-CAR, of the present invention have been introduced and expressed, against MOLT4 cells expressing CD5 and CCRF-CEM cells expressing CD5.

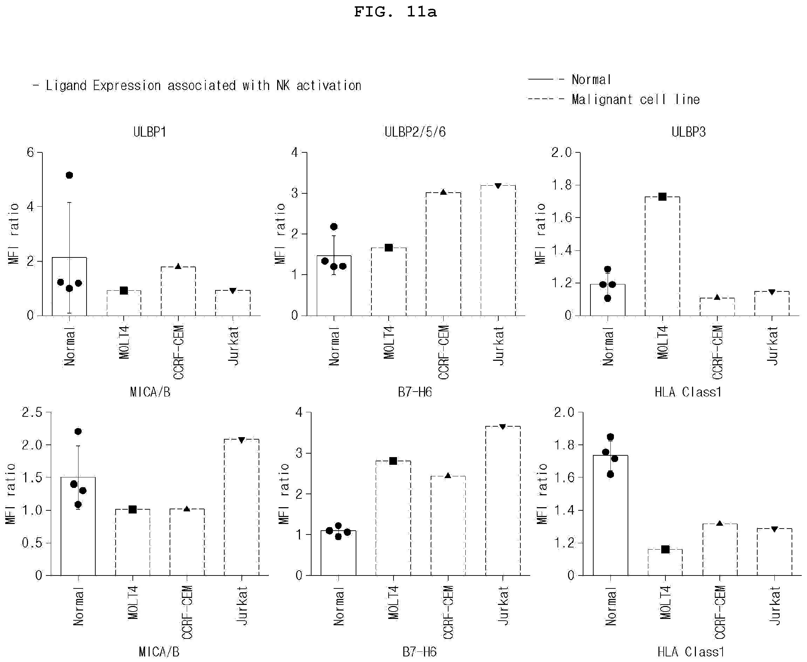

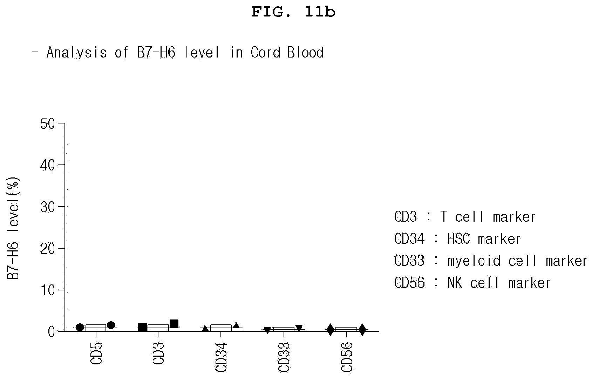

FIG. 11a shows the results obtained by confirming the expression levels of ligands involved in the activation of NK cells in normal cells and cancer cell lines expressing CD5, and FIG. 11b shows the results obtained by measuring the expression level of B7-H6 in each leukocyte using MNC.

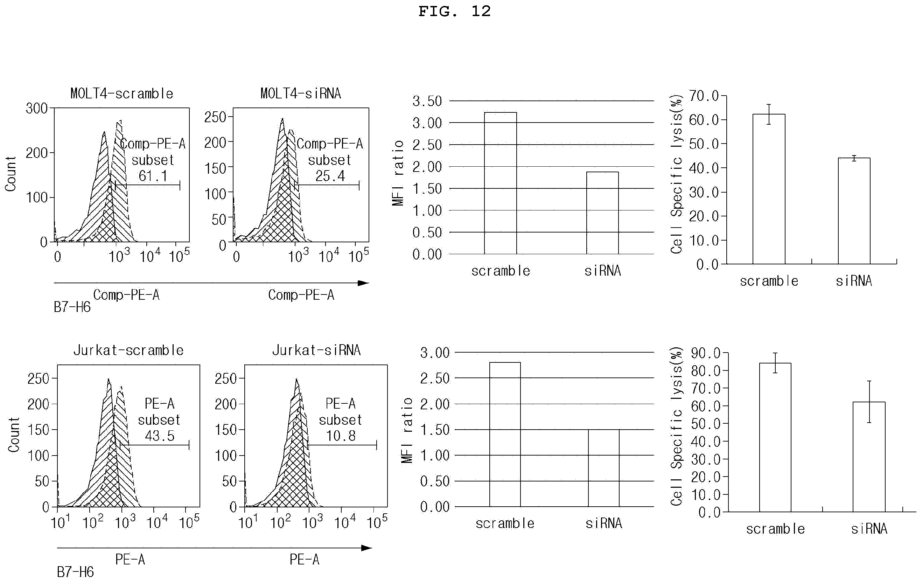

FIG. 12 is a result confirming changes in the cytotoxicity of the CD5#11-CAR-NK cells of the present invention against the cancer cell lines when the expression of B7-H6 is knocked-down in MOLT4 cells and Jurkat cells, which are cancer cell lines expressing CD5.

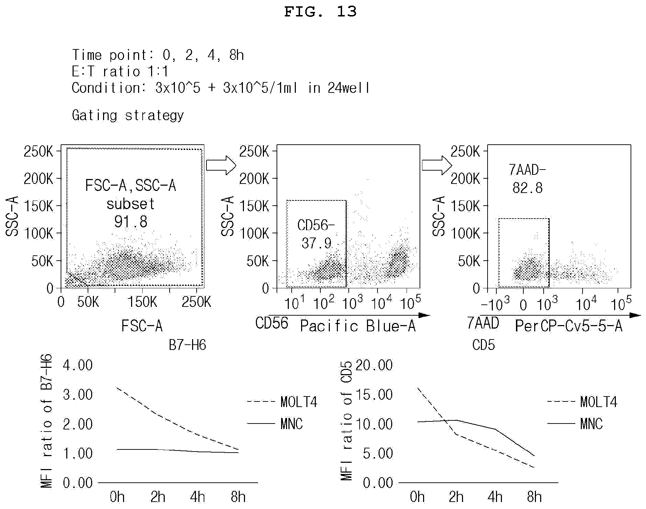

FIG. 13 shows the results obtained by measuring the expression levels of B7-H6 and CD5 over time after treating MOLT4 and MNC, which are cancer cell lines expressing CD5, with CD5#11-CARNK cells of the present invention.

## MODE FOR CARRYING OUT THE INVENTION

Since the present invention may have diverse modified embodiments, preferred embodiments below are illustrated in the drawings and are described in the detailed description of the invention. However, this does not limit the present invention within specific embodiments and it should be understood that the present invention covers all the modifications, equivalents, and replacements within the idea and technical scope of the present invention. In the following description, if a detailed description related to well-known technology is determined to obscure subject matters of the present invention, the detailed description may be omitted.

**1. Novel Anti-CD5 Antibody, Antigen-binding Fragment Thereof, and Chimeric Antigen Receptor (CAR) Including the Same, and Polynucleotide and Expression Vector for the Expression of the Same**

One aspect of the present invention, there is provided an anti-CD5 antibody and antigen-binding fragment thereof which can specifically bind to CD5.

As used herein, the term "antibody" refers to an immunoglobulin molecule having immunological reactivity by specifically binding to an epitope of an antigen. The antibody may include all of a monoclonal antibody, a polyclonal antibody, an antibody having a full-length chain structure (a full-length antibody), a functional fragment (antigen-binding fragment) having at least an antigen binding function, and a recombinant antibody, and specifically, the antibody of the present invention may be a monoclonal antibody or an antigen-binding fragment thereof. The monoclonal antibody refers to an antibody molecule of a single molecular composition obtained from substantially identical antibody population, and the monoclonal antibody exhibits single binding specificity and affinity for a specific epitope. The full-length antibody has a structure having two full-length light chains and two full-length heavy chains, and each light chain may be linked to each heavy chain through disulfide bonds. The antibody may include heavy chain (HC) and light chain (LC) polypeptides, and the heavy chain and the light chain may include a variable region and a constant region.

The constant region is a region that mediates the binding of the antibody to host tissue including various types of cells (such as T-cells) of the immune system, components of the complement system, and the like. The constant region has the same function regardless of the type of antigen as long as the antigen is the same type of antibody derived from the same species, and the amino acid sequence constituting the constant region is also identical or very similar for each antibody. The constant region may be divided into a constant region of a heavy chain (abbreviated as CH) and a constant region of a light chain (abbreviated as CL). The heavy chain constant region has gamma ($\gamma$), mu ($\mu$), alpha ($\alpha$), delta ($\delta$), and/or epsilon ($\varepsilon$) types, and has gamma 1 ($\gamma$1), gamma 2 ($\gamma$2), gamma 3 ($\gamma$3), gamma 4 ($\gamma$4), alpha 1 ($\alpha$1), and/or alpha 2 ($\alpha$2) as a subclass. The light chain constant region has kappa (k) and lambda ($\lambda$) types. IgG includes IgG1, IgG2, IgG3, and IgG4 as a subtype.

The variable region may be divided into a variable region of a heavy chain (abbreviated as VH) and a variable region of a light chain (abbreviated as VL) as an antibody region having specificity for an antigen. The variable region may include three complementary-determining regions (CDRs) and four framework regions (FRs). The CDRs may be ring-shaped sites involved in the recognition of an antigen, and specificity for the antigen may be determined according to an amino acid sequence of the CDR. The CDRs may be referred to as CDR1, CDR2, and CDR3 according to the order thereof, and may be referred to as CDR-H1, CDR-H2, and CDR-H3 for the heavy chain variable regions and CDR-L1, CDR-L2, and CDR-L3 for the light chain variable regions, according to the polypeptide type among heavy and light chains. Similarly, the FRs may be referred to as FR-H1, FR-H2, FR-H3, and FR-H4 for the heavy chain variable regions, and may be referred to as FR-L1, FR-L2, FR-L3, and FR-L4 for the light chain variable regions. In addition, the CDRs and the FRs may be arranged in the following order in each variable region.

As used herein, the term "antigen-binding fragment" refers to any fragment of the humanized antibody of the present invention that retains the antigen-binding function of the antibody. The antigen-binding fragment may be referred to as "fragment" or "antibody fragment," and the antigen-binding fragment may be Fab, Fab', F(ab')$_2$, Fv or the like, but is not limited thereto.

The Fab has a structure having variable regions of the light and heavy chains, a constant region of the light chain, and a first constant region of the heavy chain (CH1 domain). The Fab' differs from the Fab in that the Fab' has a hinge region including at least one cysteine residue at the C-terminus of the CH1 domain of the heavy chain. The F(ab')$_2$ is generated when the cysteine residue of the hinge region of the Fab' forms a disulfide bond. The Fv refers to the minimum antibody fragment with only a heavy chain variable region and a light chain variable region. The two-chain Fv may be formed by linking the heavy chain variable region and the light chain variable region through a non-covalent bond, and the single-chain Fv may be formed by linking the heavy chain variable region and the light chain variable region via a peptide linker through a covalent bond or directly linking the heavy chain variable region and the light chain variable region at the C-terminal, thereby forming a structure such as a dimer like the two-chain Fv. The antigen-binding fragment may be prepared by using a protease (for example, papain restricts the whole antibody to obtain an Fab, and pepsin restricts the whole body to obtain an F(ab')2 fragment), or through gene recombination technology, but is not limited thereto.

The linker may be a peptide linker and may have a length of about 10 to 25 amino acids. For example, the linker may include a hydrophilic amino acid such as glycine (G) and/or serine (S). The linker may include, for example, $(GS)_n$, $(GGS)_n$, $(GSGGS)_n$, or $(G_nS)_m$ (where each of n and m is 1 to 10), for example, $(G_nS)_m$ (where each of n and m is 1 to 10), but is not limited thereto.

As used herein, the term "epitope" refers to a specific site on an antigen that can be specifically recognized and bound by an immunoglobulin, an antibody, or an antigen-binding fragment thereof. The epitope may be formed from a continuous amino acid or from a discontinuous amino acid juxtaposed by a tertiary folding of a protein.

The "specifically binding" may mean binding to other molecules with a binding affinity greater than the background binding, for example, the extracellular domain may be binding to a target antigen with an affinity of about $10^{-5}$ M or greater or with Ka (equilibrium dissociation constant of a specific binding interaction with a unit of 1/M). With respect to the affinity, the equilibrium dissociation constant ($K_d$) of a specific binding interaction with a unit of M may be in a range of $10^{-5}$ M to $10^{-13}$ M or may be equal to or less than the range.

The antibody or antigen-binding fragment thereof includes: a heavy chain variable region including i) a heavy chain CDR1 having any one amino acid sequence selected from the group consisting of SEQ ID NO: 2, SEQ ID NO: 10, SEQ ID NO: 18, and SEQ ID NO: 26, ii) a heavy chain CDR2 having any one amino acid sequence selected from the group consisting of SEQ ID NO: 3, SEQ ID NO: 11, SEQ ID NO: 19, and SEQ ID NO: 27, and iii) a heavy chain CDR3 having any one amino acid sequence selected from the group consisting of SEQ ID NO: 4, SEQ ID NO: 12, SEQ ID NO: 20, and SEQ ID NO: 28; and a light chain variable region including iv) a light chain CDR1 having any one amino acid sequence selected from the group consisting of SEQ ID NO: 6, SEQ ID NO: 14, SEQ ID NO: 22, and SEQ ID NO: 30, v) a light chain CDR2 having any one amino acid sequence selected from the group consisting of SEQ ID NO: 7, SEQ ID NO: 15, SEQ ID NO: 23, and SEQ ID NO: 31, and vi) a light chain CDR3 having any one amino acid sequence selected from the group consisting of SEQ ID NO: 8, SEQ ID NO: 16, SEQ ID NO: 24, and SEQ ID NO: 32, and specifically binds to CD5.

The heavy chain variable region may have an amino acid sequence of SEQ ID NO: 5, SEQ ID NO: 13, SEQ ID NO: 21, or SEQ ID NO: 29.

The light chain variable region may have an amino acid sequence of SEQ ID NO: 9, SEQ ID NO: 17, SEQ ID NO: 25, or SEQ ID NO: 33.

The light chain variable region may have an amino acid sequence of SEQ ID NO: 8 or SEQ ID NO: 16.

The antibody or antigen-binding fragment thereof of the present invention may further include, for example, a heavy chain constant region and/or a light chain constant region of an antibody derived from a human, and the heavy chain constant region and/or the light chain constant region of the antibody derived from a human may be used without limitation in its type or amino acid sequence so long as the antibody or antigen-binding fragment thereof does not inhibit the characteristic of specifically binding to CD5.

The amino acid sequences as described above may include a variant having a different sequence by deletion, insertion, substitution, or a combination thereof of amino acid residues within a range that does not affect the structure, function, or activity of a polypeptide including the amino acid sequences. In addition, the amino acid sequences may include an amino acid which is typically modified known in the art, and the amino acid modification may include, for example, phosphorylation, sulfation, acrylation, glycosylation, methylation, farnesylation, or the like. The humanized antibody or antigen-binding fragment thereof of the present invention includes not only the above-described amino acid sequences, but also has substantially the same amino acid sequences as the above-described amino acid sequences, or includes variants thereof. The meaning of having substantially the same amino acid sequence may include an amino acid sequence having at least 90%, at least 91%, at least 92%, at least 93%, at least 94%, at least 95%, at least 96%, at least 97%, at least 98%, at least 99%, or at least 99.5% homology with the above-described amino acid sequence, but is not limited thereto.

As used herein, the term "chimeric antigen receptor (CAR)" refers to a synthetic complex designed to induce an immune response against a target antigen and a cell expressing the antigen when the target antigen and the cell expressing the antigen are recognized and bound. The chimeric antigen receptor may include an extracellular binding domain, a transmembrane domain, and an intracellular signaling domain. The chimeric antigen receptor is expressed on the surface of the immune cell, and recognizes and binds to a specific antigen, for example, an antigen specifically expressed on the surface of cancer cell, through an antigen-binding site included in the extracellular domain, thereby triggering a signaling pathway in the immune cell and changing the activity of the immune cell, and thus inducing an immune response targeting only the specific antigen.

The chimeric antigen receptor (CAR) of the present invention includes: an extracellular binding domain including an antigen-binding site that specifically binds to CD5 (ephrin type-A receptor 2); a transmembrane domain; and an intracellular signaling domain.

The antigen-binding site specifically binding to CD5 may be a single chain variable fragment (scFv) of an anti-CD5 antibody including: a heavy chain variable region including i) a heavy chain CDR1 having any one amino acid sequence selected from the group consisting of SEQ ID NO: 2, SEQ ID NO: 10, SEQ ID NO: 18, and SEQ ID NO: 26, ii) a heavy chain CDR2 having any one amino acid sequence selected from the group consisting of SEQ ID NO: 3, SEQ ID NO: 11, SEQ ID NO: 19, and SEQ ID NO: 27, and iii) a heavy chain CDR3 having any one amino acid sequence selected from the group consisting of SEQ ID NO: 4, SEQ ID NO: 12, SEQ ID NO: 20, and SEQ ID NO: 28; and a light chain variable region including iv) a light chain CDR1 having any one amino acid sequence selected from the group consisting of SEQ ID NO: 6, SEQ ID NO: 14, SEQ ID NO: 22, and SEQ ID NO: 30, v) a light chain CDR2 having any one amino acid sequence selected from the group consisting of SEQ ID NO: 7, SEQ ID NO: 15, SEQ ID NO: 23, and SEQ ID NO: 31, and vi) a light chain CDR3 having any one amino acid sequence selected from the group consisting of SEQ ID NO: 8, SEQ ID NO: 16, SEQ ID NO: 24, and SEQ ID NO: 32.

When the chimeric antigen receptor of the present invention is expressed on the surface of immune cell, if CD5, a target antigen, binds to the receptor, a signaling pathway is triggered in the immune cell, and the improvement of cytotoxicity (or cytolytic activity) of the immune cell and/or the promotion of cytokine secretion of the immune cell may be induced. The improvement of cytotoxicity (or cytolytic activity) or the promotion of cytokine secretion may mean exhibiting the higher level of cytotoxicity (or cytolytic activity) or cytokine secretion than the level of cytotoxicity (or cytolytic activity) or cytokine secretion exhibited by immune cells in which antigens are not present.

The extracellular domain may further include at least one selected from the group consisting of a hinge domain and a spacer domain. The antigen-binding site of the extracellular domain may be linked to the transmembrane domain through the hinge domain and/or the spacer domain.

The hinge domain is a part that enables the antigen binding site to be physically spaced apart from the surface of the immune cell on which the chimeric antigen receptor is expressed, so as to allow appropriate cell/cell contact, the binding of an appropriate antigen/antigen binding site, and the activation of an appropriate chimeric antigen receptor, and may play an important role in determining the position of the extracellular domain. The chimeric antigen receptor may include one or more hinge domains between the extracellular domain and the transmembrane domain. The hinge domain may be derived from a natural, synthetic, semi-synthetic or recombinant source. The hinge domain may include an amino acid sequence of a naturally occurring immunoglobulin hinge region or an altered immunoglobulin hinge region. The altered hinge region refers to (a) the naturally occurring hinge region having at most 30% amino acid change (e.g., at most 25%, 20%, 15%, 10% or 5% amino acid substitution or deletion), (b) a portion of the naturally occurring hinge region having a length of at least 10 amino acids (e.g., at least 12, 13, 14 or 15 amino acids) having at most 30% amino acid change (e.g., at most 25%, 20%, 15%, 10% or 5% amino acid substitution or deletion), or (c) a portion of the naturally occurring hinge region including a core hinge region (which may be a length of 4, 5, 6, 7, 8, 9, 10, 11, 12, 13, 14 or 15 amino acids, or at least 4, 5, 6, 7, 8, 9, 10, 11, 12, 13, 14 or 15 amino acids). In a certain embodiment, one or more cysteine residues in the naturally occurring immunoglobulin hinge region may be substituted with one or more other amino acid residues (e.g., one or more serine residues). The altered immunoglobulin hinge region may alternatively or additionally have another amino acid residue, such as the proline residue of the wild-type immunoglobulin hinge region substituted with cysteine. The hinge domain may be a hinge region derived from an extracellular domain of a type 1 membrane protein such as CD8, CD4, CD28, and CD7, but any hinge domain may be used without limitation as long as it can link the antigen binding site, the transmembrane domain, and the intracellular signaling domain through the cell membrane. The hinge domain may also be the wild-type hinge region from these molecules or be altered.

The spacer domain may be referred to as a linking domain, may include, for example, a CD28-derived hinge domain and/or a CD8-derived hinge domain, and may include the whole or a part of the CD28-derived hinge domain and/or the CD8-derived hinge domain.

The hinge domain and/or the spacer domain may be at least one selected from the group consisting of a Myc epitope, a CD8 hinge domain, and Fc, and specifically, may include a Myc epitope and a CD8 hinge domain.

The transmembrane domain refers to a partial region that serves to link and fuse the extracellular domain and the intracellular signaling domain to each other and to fix the chimeric antigen receptor to the plasma membrane of the immune cell. The transmembrane domain may be derived from a natural, synthetic, semi-synthetic, or recombinant source. The transmembrane domain may be any one selected from the group consisting of an alpha ($\alpha$), beta ($\beta$), or zeta ($\zeta$) chain of a T-cell receptor (TCR), CD28, CD3 epsilon ($\varepsilon$), CD45, CD4, CD5, CD8, CD9, CD16, CD22, CD33, CD37, CD64, CD80, CD86, CD134, CD137, and CD154, but is not limited thereto.

The transmembrane domain may be attached to the extracellular domain via a linker. For example, the linker may be a short oligopeptide or polypeptide linker having a length of 2 to 10 amino acids, for example, a glycine (G)-serine (S) doublet, but is not limited thereto.

The intracellular signaling domain corresponds to a portion that serves to deliver signals generated according to the binding of the chimeric antigen receptor and the antigen into the immune cell in order to induce the function of the immune cell (e.g., the activation including the release of cytotoxic (or cytolytic activity) factors to the target cell in which the chimeric antigen receptor and antigen are bound, cytokine production, proliferation and cytotoxic or cytolytic activity, or other cellular response caused by the antigen binding). The intracellular signaling domain may be a part of a protein that transmits an effector function signal and directs the cell to perform a special function.

As the intracellular signaling domain, an intracellular signaling domain which has been used in the development process of the existing chimeric antigen receptor may be used. Specifically, it may include only CD3$\zeta$ used in the first-generation chimeric antigen receptor (CAR). In addition, as used in the second-generation CAR, the form in which a co-stimulatory domain (CD28 or CD137/4-1BB) and CD3$\zeta$ are bound may be used to improve the reactivity to immune cells. In addition, two or more co-stimulatory domains may be used as used in the third generation CAR, and in this case, the co-stimulatory domains may be bound to 4-1BB, CD28, or OX40, and the like, to achieve the extension and persistence of immune cells including the CAR *in vivo.* Further, as used in the fourth generation CAR, additional genes encoding cytokines such as IL-12 or IL-15 may be included so that the CAR-based immunoproteins of the cytokines may be further expressed, and as used in the fifth generation CAR, interleukin receptor chains, for example, IL-2R$\beta$ may

be further included to enhance immune cells.

The intracellular signaling domain may include at least one selected from the group consisting of T-cell receptor (TCR) zeta (ζ), FcR gamma (γ), FcR beta (β), CD3 gamma (γ), CD3 delta (δ), CD3 epsilon (ε), CD3 zeta (ζ), CD5, CD22, CD79a, CD79b, and CD66d.

More specifically, the activation of the immune cell by the intracellular signaling domain may be mediated by two different classes of intracellular signaling domains. For example, the immune cell activation may be mediated by a primary signaling domain that initiates antigen-dependent primary activation and a co-stimulatory signaling domain that acts in an antigen-independent manner to provide a secondary signal. Accordingly, the intracellular signaling domain may include a primary signaling domain and a co-stimulatory signaling domain.

The primary signaling domain refers to a signaling domain that regulates the immune cell activation in a stimulating or inhibiting manner. The primary signaling domain acting in a stimulating manner may contain a signaling motif known as an immunoreceptor tyrosine-based activation motif or ITAM. The ITAM containing the primary signaling domain may be TCRζ, FcRγ, FcRβ, CD3γ, CD3δ, CD3ε, CD3ζ, CD5, CD22, CD79a, CD79b, CD66d, or the like, but is not limited thereto. More specifically, the primary signaling domain may be CD3ζ (zeta), but is not limited thereto.

The co-stimulatory signaling domain refers to an intracellular signaling domain of a co-stimulatory molecule. The co-stimulatory signaling domain may include a co-stimulatory signaling domain selected from the group consisting of CD2, CD7, CD27, CD28, CD30, CD40, 4-1BB (CD137), OX40 (CD134), CDS, ICAM-1, ICOS (CD278), LFA-1 (CD11a/CD18), GITR, MyD88, DAP10, DAP12, PD-1, LIGHT, NKG2C, CD5, CD83, and the like, but is not limited thereto. Specifically, the costimulating molecule may be DAP10, but is not limited thereto.

The chimeric antigen receptor may include two or more intracellular signaling domains, and when the chimeric antigen receptor includes two or more intracellular signaling domains, the intracellular signaling domains may be linked to each other in tandem. Alternatively, the intracellular signaling domains may be linked via a polypeptide linker consisting of 2 to 10 amino acids, and the linker sequence may be, for example, a glycine-serine continuous sequence. The linker may include, for example, $(GS)_n$, $(GGS)_n$, $(GSGGS)_n$, or $(G_nS)_m$ (where each of n and m is 1 to 10), for example, $(G_nS)_m$ (where each of n and m is 1 to 10), but is not limited thereto.

The chimeric antigen receptor may further include an immune function promoting factor of an immune cell, and for example, the immune function promoting factor of the immune cell may be an interleukin signal sequence. The interleukin signal sequence may be characterized by inducing the expression of interleukin (IL)-12, IL-8, IL-2, and the like, but is not limited thereto. In addition, when the immune cell is a T cell, the immune function promoting factor may be IL-7, CCL19, or the like, but is not limited thereto.

Meanwhile, the chimeric antigen receptor may further include a signal peptide for domain exposure. The signal peptide may be a type of secreted or transmembrane protein, which may direct the chimeric antigen receptor to be transported to the cell membrane or cell surface, and provide accurate positioning. A specific type may be a CD8 alpha or mouse light chain kappa signal peptide, but is not limited thereto.

In one specific embodiment of the present invention, the chimeric antigen receptor (CD5-CAR) according to the present invention was prepared by designing a chimeric antigen receptor in the form in which the scFv of the anti-CD5 antibody of four types (#1, #4, #11, and #14) having the amino acid sequence as described above was included in the extracellular domain, the CD28 linked via the Myc and hinge domains was included in the transmembrane domain and the intracellular signaling domain, and the CD3-zeta and DAP10 were linked to the intracellular signaling domain. As described above, when the chimeric antigen receptor of the present invention is expressed on the surfaces of immune cell and is bound by recognizing CD5, the chimeric antigen receptor may induce to improve the cytotoxicity or cytolytic activity of immune cell or promote the cytokine secretion of immune cell. Therefore, if the antigen-binding site of the chimeric antigen receptor recognizes and binds to an antigen when cancer cells expressing CD5 are present, the improvement in the cytotoxic or cytolytic activity of immune cells and/or the promotion of cytokine secretion may be induced by the signaling pathway, and thus the chimeric antigen receptor may be effectively used as a chimeric antigen receptor having excellent cytotoxic effect or cytolytic effect for attacking cancer cells.

Another aspect of the present invention, there is provided a polynucleotide and an expression vector for expressing the chimeric antigen receptor.

The polynucleotide includes a base sequence encoding the chimeric antigen receptor.

As used herein, the term "polynucleotide" encompasses DNA (gDNA and cDNA) and RNA molecules, and nucleotides, which are basic structural units, include not only natural nucleotides, but also analogs having modified sugar or base sites.

The encoding of the chimeric antigen receptor means that genetic information, through which a protein having an amino acid sequence of the chimeric antigen receptor of the present invention may be synthesized by a general protein expression process such as transcription and translation of the polynucleotide, is encoded. In this case, a polynucleotide encoding not only a protein having an amino acid sequence that is completely identical to that of the chimeric antigen receptor, but also a protein having an amino acid sequence that is substantially identical to the protein as described above, or a protein having activity that is identical and/or similar to the protein, may be included in the scope of the present invention.

Specifically, the polynucleotide of the present invention may include a base sequence encoding an antigen-binding fragment of an anti-CD5 antibody that specifically binds to the CD5.

In addition, the polynucleotide of the present invention may include not only the base sequence encoding the antigen binding fragment, but also a base sequence encoding other extracellular domain portions linked thereto, the transmembrane domain, and/or the intracellular signaling domain.

The description of the chimeric antigen receptor, such as the antibody, antigen-binding fragment, extracellular domain, transmembrane domain, and intracellular signaling domain, is the same as described above.

The polynucleotide of the present invention may include a base sequence substantially identical to the base sequences listed above. The substantially identical base sequence may include, for example, the case in which the same amino acid may be synthesized when transcribed and translated, and may be a base sequence having at least 90%, at least 91%, at least 92%, at least 93%, at least 94%, at least 95%, at least 96%, at least 97%, at least 98%, at least 99%, or at least 99.5% homology with the listed base sequences, but is not limited thereto.

The polynucleotide encoding the chimeric antigen receptor may include a base sequence optimized according to the type of the organism into which the polynucleotide will be introduced and expressed, and an expression system such as transcription and translation of the organism. This is due to the degeneracy of the codon, and accordingly, there may be various combinations of nucleotide sequences capable of encoding the protein expressed thereby, all of which are included within the scope of the present invention. The modification of the polynucleotide according to the codon optimization may be determined according to the kind of organisms in which the chimeric antigen receptor of the present invention is expressed and applied, and for example, the polynucleotide of the present invention may be a polynucleotide modified by being optimized for the selection of a codon of a mammal or a primate, and more specifically, may be modified by being optimized to be suitable for the expression and action from a human.

The expression vector of the present invention includes the polynucleotide.

Since the polynucleotide includes a base sequence encoding the chimeric antigen receptor of the present invention, the expression vector including the polynucleotide may be used to express and prepare the chimeric antigen receptor, and may serve to transfer the polynucleotide to a specific cell or organism or may be used to keep and store the polynucleotide so that the chimeric antigen receptor may be expressed.

The term "vector" refers to a DNA construct containing a DNA sequence operably linked to a suitable regulatory sequence capable of expressing DNA in a suitable host. The vector may be a plasmid, a phage particle, or simply a potential genomic insert. Once transformed into a suitable host, the vector may replicate itself and function regardless of the host genome, or in some cases, the vector may be integrated into the genome itself. In addition, the term "expression vector" generally means a double-stranded DNA fragment functioning as a recombinant carrier into which a xenogeneic DNA fragment is inserted. Here, the xenogeneic DNA refers to a heterologous DNA, which is not naturally found in a host cell. The expression vector may be selfreplicable regardless of host chromosomal DNA once in a host cell, and may produce several copies of the vector and (xenogeneic) DNA inserted thereinto.

The expression vector may be constructed using a prokaryotic cell or a eukaryotic cell as a host.

For example, when the expression vector uses a prokaryotic cell as a host, it generally carries a strong promoter to initiate transcription (e.g., tac promoter, lac promoter, lacUV5 promoter, lpp promoter, pLλ promoter, pPλ promoter, rac5 promoter, amp promoter, recA promoter, SP6 promoter, trp promoter, T7 promoter, or the like), a ribosome binding site for translation initiation, and a transcription/translation termination sequence. When *E. coli* (e.g., HB101, BL21, DH5α, or the like) is used as a host cell, the promoter and operator region for the tryptophan biosynthesis pathway (Yanofsky, C, J Bacteriol, (1984) 158:1018-1024) and the leftward promoter from phage λ (pLλ promoter; Herskowitz, I and Hagen, D, Ann Rev Genet, (1980) 14:399-445) may be used as regulating sequences. When *Bacillus* sp. is used as a host cell, a promoter of toxin protein gene of *Bacillus thuringiensis* (Appl Environ Microbiol (1998) 64:3932-3938; Mol Gen Genet (1996) 250:734-741) or any promoters expressible in *Bacillus* sp. may be used as regulating sequences. The expression vector may be prepared by manipulating a plasmid (*e.g.*, pCL, pSC101, pGV1106, pACYC177, ColE1, pKT230, pME290, pBR322, pUC8/9, pUC6, pBD9, pHC79, pIJ61, pLAFR1, pHV14, pGEX series, pET series, pUC19 and the like), a phage (e.g., λgt4λB, λ-Charon, λΔz1, M13 and the like) or a virus (*e.g.,* SV40, etc.), which is often used in the art.

When the expression vector uses an eukaryotic cell as a host, promoters derived from a genome of mammal cells (*e.g.*, a metallothionin promoter, a β-actin promoter, a human hemoglobin promoter, and a human muscle creatine promoter) or promoters derived from mammal viruses (*e.g.*, an adenoviral late promoter, a vaccinia virus 75K promoter, an SV40 promoter, a cytomegalovirus (CMV) promoter, a tk promoter of HSV, a mouse mammary tumor virus (MMTV) promoter, an LTR promoter of HIV, a promoter of Moloney virus, a promoter of Epstein-barr virus (EBV), and a promoter of Rous sarcoma virus (RSV)) may be used, and the expression vector may generally have a polyadenylation sequence as a transcription termination sequence. The expression vector may have the CMV promoter.

In addition, the expression vector may be fused with another sequence to facilitate the purification of the antibody expressed therefrom. The fused sequences include, for example, glutathione S-transferase (Pharmacia, USA), maltose binding protein (NEB, USA), FLAG (IBI, USA), 6xHis (hexahistidine; Quiagen, USA) and the like. In addition, since the protein expressed by the expression vector of the present invention is a chimeric antigen receptor, the expressed protein may be easily purified through a protein A column or the like without an additional sequence for purification in consideration of characteristics thereof.

The expression vector includes an antibiotic resistance gene conventionally used in the art as a selectable marker, and may include, for example, resistance genes against ampicillin, gentamicin, carbenicillin, chloramphenicol, streptomycin, kanamycin, geneticin, neomycin and tetracycline.

In addition, the expression vector may further include an enhancer that may be used to promote and regulate the expression, as well as a promoter for promoting the expression of a gene to be transfected, and basal elements required for the transcription.

## 2. Immune Cells Expressing Chimeric Antigenic Receptors for CD5 on the Surfaces Thereof

Another aspect of the present invention, there is provided immune cells that exhibit improved cytotoxicity or cytolytic activity against cancer cells expressing CD5.

The immune cells express the chimeric antigen receptor of the present invention as described above on the surfaces thereof. The chimeric antigen receptor is the same as described above in **"1. *Novel Anti-CD5 Antibody, Antigen-binding Fragment Thereof, and Chimeric Antigen Receptor (CAR) Including the Same, and Polynucleotide and Expression Vector for the Expression of the Same.*"** Specifically, the chimeric antigen receptor may include an antigen binding site capable of specifically binding to the CD5 antigen expressed in the cancer cells.

The "expression" of the chimeric antigen receptor on the surface of the immune cell means that any immune cell is engineered by the addition or modification of a nucleic acid encoding the chimeric antigen receptor. Accordingly, in order to express the chimeric antigen receptor on the surface of the immune cell as described above, a polynucleotide encoding the chimeric antigen receptor or an expression vector including the polynucleotide may be transfected or transduced into the immune cell. The transfection may be performed by various methods known in the art, such as calcium phosphate-DNA co-precipitation, DEAE-dextran-mediated transfection, polybrene-mediated transfection, electroporation, microinjection, liposome-mediated transfection, liposome fusion, lipofectamine, and protoplast fusion. In addition, the transfection refers to a delivery of a gene into the cell by using a virus or viral vector particle by means of infection. In the present specification, the transfection and the transduction may be used interchangeably, and preferably interpreted, in a wider sense, as transformation by delivering a heterologous gene in a host cell, and the cell into which the heterologous gene is introduced by transfection or transduction is referred to as a transformant.

The immune cell may be used without limitation as long as the immune cell is capable of inducing immunity and resulting in a desired therapeutic effect, may be obtained from peripheral blood, umbilical cord blood, bone marrow, tumor infiltrating lymphocytes, lymph node tissue, or thymus gland tissue, and may be obtained by differentiating cells from placenta cells, embryonic stem cells, induced pluripotent stem cells, or hematopoietic stem cells. In addition, the immune cell may be obtained from human, monkey, chimpanzee, dog, cat, mouse, rat, and gene-inserted species thereof as well as from an established cell line.

The method for obtaining immune cells may use any means known in the art, and the immune cells may be obtained from autologous, allogeneic, or xenogeneic species. The term "autologous" refers to all cells derived from the same individual as the individual to which the cells are reintroduced later, the term "allogeneic" refers to all cells derived from other animals of the same species as the individual to which the cells are introduced, and "xenogeneic" refers to cells derived from animals of other species.

In particular, the immune cells may be any one selected from natural killer cells (NK cells), T cells, natural killer T cells (NKT cells), cytokine induced killer cells (CIK), macrophages, dendritic cells, and the like, but are not limited thereto. Accordingly, the immune cells expressing the chimeric antigen receptors according to the present invention on the cell surfaces may be chimeric antigen receptor natural killer cells (CAR-NK cells), chimeric antigen receptor T cells (CAR-T cells), chimeric antigen receptor natural killer T cells (CAR-NKT cells), chimeric antigen receptor macrophages (CAR-macrophages), or the like.

The immune cells provided by the present invention may adjust the initiation (on)/the stopping (off) of a reaction with target cells of a conventional chimeric antigen receptor, and thus include a safety switch which may be very beneficial in situations requiring subsequent cell therapy and the activity of therapeutic cells to be increased or decreased. For example, when the immune cells expressing the chimeric antigen receptors are provided to a patient, there may be adverse effects in some situations, such as off-target toxicity. Alternatively, for example, the therapeutic cells may act to reduce the number of tumor cells or tumor size, and may no longer be needed. In such a situation, it is possible to regulate so that the therapeutic cells are no longer activated. In particular, the CAR-NK cells obtained by introducing chimeric antigen receptors into natural killer cells have the advantages that the CAR-NK cells may be utilized as a general-purpose therapeutic agent by targeting various cancer cells, as well as may solve limitations caused by continuous toxicity, risk of autoimmune diseases, limitations of graft-versus-host disease (GVHD) for xenograft transplantation, limitations of non-target toxicity, and the like, which are present in cancer immunotherapy when using a conventional CAR-T therapeutic agent based on T cells, through the on/off switch of reaction.

The immune cell of the present invention expresses a chimeric antigen receptor on the cell surface, the chimeric antigen receptor including, as an extracellular domain, an antigen-binding variable fragment (scFv) of an antibody capable of specifically recognizing and binding to CD5 which may be specifically expressed in cancer cells. Therefore, when the cancer cells expressing CD5 are present, a signaling pathway may be triggered by the chimeric antigen receptor, thereby further improving the cytotoxicity or cytolytic activity of immune cells and increasing the amount of cytokines secreted. Thus, the immune cells of the present invention may have the activity of attacking and treating cancer cells.

In a specific embodiment of the present invention, the chimeric antigen receptor including an antigen-binding variable fragment of four CD5-specific antibodies of the present invention as an extracellular domain was expressed on the surface of a natural killer cell. Each of the four natural killer cells was found to have improved cytotoxicity (or cytolytic activity) when co-cultured with MOLT4 cells, CCRF-CEM cells, Jurkat cells, or the like, which are cancer cell lines expressing CD5, and increased secretion and degranulation of cytokines, as well as exhibit decreased cytotoxicity against MNCs including normal cells expressing CD5, and thus it was confirmed that the immune cells of the present invention have an excellent effect of selectively treating cancer cells expressing CD5.

### 3. Use of Immune Cells of the Present Invention for Preventing or Treating Cancer

Still another aspect of the present invention, there is provided a pharmaceutical composition for preventing or treating cancer, the pharmaceutical composition including the immune cells as an active ingredient.

The description of the immune cells, the chimeric antigen rectors expressed therefrom, or the like is the same as described in **"1. *Novel Anti-CD5 Antibody, Antigen-binding Fragment Thereof, and Chimeric Antigen Receptor (CAR) Including the Same, and Polynucleotide and Expression Vector for the Expression of the Same,"*** and **"2. *Immune Cells Expressing Chimeric Antigenic Receptors for CD5 on the Surfaces Thereof,"*** and thus the description will be omitted in order to avoid repetition.

As used herein, the terms "cancer" and "tumor" are used in the same sense, and typically refer to or describe a physiological condition of mammals characterized by cell growth/proliferation that is not regulated.

The cancer or carcinoma which may be treated with the pharmaceutical composition of the present invention is not particularly limited, and includes both solid cancer and blood cancer. In particular, the cancer may be blood cancer such as leukemia or lymphoma, and specifically, may be selected from among thymic carcinoma, non-Hodgkin lymphoma, diffuse large cell lymphoma, small lymphocytic lymphoma, T-cell neoplasm, peripheral T-cell lymphoma, mantle cell lymphoma, T-cell acute lymphoblastic leukemia, chronic lymphocytic leukemia, and the like.

In addition, the pharmaceutical composition of the present invention may include 1 to 10 times, 2 to 10 times, or 5 to 8 times the number of immune cells as compared to the number of tumor cells of an individual to be treated, but is not limited thereto.

Meanwhile, it is common to use chemotherapy in combination with radiotherapy for cancer treatment. Anticancer agents used in chemotherapy induce apoptosis of cells with active proliferation, and radiation irradiation to increase the therapeutic effect of anticancer agents increases such apoptosis. In this case, the cells in which apoptosis is induced by the anticancer agents and radiation are not limited to cancer cells, and may also have an effect on immune cell therapy administered to an individual for immunotherapy.

The chemotherapy includes, but is not limited to, CHOP (cyclophosphamide, doxorubicin, vincristine, prednisone), EPOCH (etoposide, vincristine, doxorubicin, cyclophosphamide, prednisone), or any other multi-drug regimens.

As used herein, the term "cellular therapeutic agent" refers to a drug used for the purpose of treatment, diagnosis and prevention in cells and tissues produced by separation from an individual, culture and specialized manipulation (U.S. FDA regulations), and to a drug used for treatment, diagnosis and prevention through a series of actions, such as proliferation and selection of living autologous, allogeneic, or xenogeneic cells *in vitro,* or otherwise altering the biological characteristics of the cells in order to restore the function of the cells or tissue.

Meanwhile, the pharmaceutical composition of the present invention may further include a pharmaceutically or pharmacologically acceptable carrier. The term "pharmaceutically acceptable" means that the carrier does not have toxicity equal to or more than the toxicity to which a subject to be applied (prescribed) is adaptable without inhibiting the activity of the active ingredient, and the "carrier" is defined as a compound that facilitates the addition of the compound into cells or tissue.

The pharmaceutical composition of the present invention may be administered alone or in combination with any convenient carrier, and the like, and the dosage form may be a single-dose unit or multiple-dose unit. The pharmaceutical composition may be a solid formulation or a liquid formulation. The solid formulation includes, but is not limited to, a powder, a granule, a tablet, a capsule, a suppository, and the like. The solid formulation may include, but is not limited to, a carrier, a flavor, a binder, a preservative, a disintegrant, a lubricant, a filler, and the like. The liquid formulation includes water, a solution such as a propylene glycol solution, a suspension, an emulsion, and the like, but is not limited thereto, and may be prepared by adding suitable colorants, flavors, stabilizers, thickeners, and the like. For example, a powder may be prepared by simply mixing a tri-hydroxy derivative of a polysaturated fatty acid, which is the active ingredient of the present invention, with a suitable pharmaceutically acceptable carrier such as lactose, starch, or microcrystalline cellulose. A granule may be prepared by mixing the tri-hydroxy derivative of the polysaturated fatty acid of the present invention, a suitable pharmaceutically acceptable carrier, and a suitable pharmaceutically acceptable binder such as polyvinylpyrrolidone or hydroxypropyl cellulose, and then utilizing wet granulation using a solvent such as water, ethanol, or isopropanol, or dry granulation using a compressive force. Also, a tablet may be prepared by mixing the granule with a suitable pharmaceutically acceptable lubricant such as magnesium stearate, and then tableting the mixture using a tableting machine.

The pharmaceutical composition of the present invention may be administered in the form of oral formulation, injectable formulation (for example, intramuscular, intraperitoneal, intravenous, infusion, subcutaneous, implant), inhalable, intranasal, vaginal, rectal, sublingual, transdermal, topical, etc. depending on the disorders to be treated and the individual's conditions, but is not limited thereto. The composition of the present invention may be formulated in a suitable dosage unit formulation including a pharmaceutically acceptable and non-toxic carrier, additive and vehicle, which are generally used in the art, depending on the routes to be administered.

In particular, the pharmaceutical composition of the present invention may be used through an injectable ampule. The injectable ampule may be prepared by being mixed with an injectable solution immediately before use, and as the injectable solution, physiological saline, glucose, mannitol, Ringer's solution, or the like may be used. The pharmaceutical composition or formulation of the present invention prepared as described above may be administered in the form of a mixture together with cells used for transplantation and other applications by using an administration method commonly used in the art. The actual dose of the active ingredient is to be determined in light of several related factors, such as the disease to be treated, the severity of the disease, the route of administration, the patient's weight, age, and sex.

In addition, the preferred dosage of the pharmaceutical composition of the present invention varies with the condition and body weight of a patient, the severity of the disease, the form of drug, the route and duration of administration, but may be appropriately selected by a person skilled in the art. For example, the pharmaceutical composition may be administered at a daily dose of about 0.0001 mg/kg to about 10 g/kg, specifically, about 0.001 mg/kg to about 1 g/kg. However, the dosage may vary with the degree of purification of the mixture, the condition of a patient (age, sex, body weight, etc.), the severity of the condition being treated, and the like. For convenience, a total daily dose of the pharmaceutical composition may be administered in multiple doses a day as needed.

Meanwhile, the composition may be in the form of a quasi-drug composition, a health food composition, or the like in addition to the pharmaceutical composition.

In addition, another aspect of the present invention, there is provided a method for preventing or treating cancer, the method including administering the immune cells to an individual in need thereof.

The immune cells may be in the form of the above-described pharmaceutical composition, and a person skilled in the art to which the present invention pertains may determine an appropriate route and dose of the pharmaceutical composition.

Hereinafter, the present disclosure will be described in more detail with reference to Examples.

However, the following examples are for illustrating the present invention, and the scope of the present invention is not limited to the following examples.

**[Example 1]**

**Preparation of Immune Cells Expressing Chimeric Antigenic Receptors for CD5 on the Surfaces Thereof**

**[1-1] Preparation of Single Chain Variable Fragment of Antibody Specifically Binding to CD5**

Four types of single chain variable fragments (scFv) (#1, #4, #11, and #14) were prepared by using sequences that play an important role in the specific binding among the anti-CD5 antibody sequences capable of specifically binding to CD5.

As shown in Table 1 below, scFv #1 was designed to include a heavy chain variable region including a heavy chain CDR1 having an amino acid sequence of SEQ ID NO: 2, a heavy chain CDR2 having an amino acid sequence of SEQ ID NO: 3, and a heavy chain CDR3 having an amino acid sequence of SEQ ID NO: 4, and a light chain variable region including a light chain CDR1 having an amino acid sequence of SEQ ID NO: 6, a light chain CDR2 having an amino acid sequence of SEQ ID NO: 7, and a light chain CDR3 having an amino acid sequence of SEQ ID NO: 8. scFv #4 was designed to include a heavy chain variable region including a heavy chain CDR1 having an amino acid sequence of SEQ ID NO: 10, a heavy chain CDR2 having an amino acid sequence of SEQ ID NO: 11, and a heavy chain CDR3 having an amino acid sequence of SEQ ID NO: 12, and a light chain variable region including a light chain CDR1 having an amino acid sequence of SEQ ID NO: 14, a light chain CDR2 having an amino acid sequence of SEQ ID NO: 15, and a light chain CDR3 having an amino acid sequence of SEQ ID NO: 16. scFv #11 was designed to include a heavy chain variable region including a heavy chain CDR1 having an amino acid sequence of SEQ ID NO: 18, a heavy chain CDR2 having an amino acid sequence of SEQ ID NO: 19, and a heavy chain CDR3 having an amino acid sequence of SEQ ID NO: 20, and a light chain variable region including a light chain CDR1 having an amino acid sequence of SEQ ID NO: 22, a light chain CDR2 having an amino acid sequence of SEQ ID NO: 23, and a light chain CDR3 having an amino acid sequence of SEQ ID NO: 24. In addition, scFv #14 was designed to include a heavy chain variable region including a heavy chain CDR1 having an amino acid sequence of SEQ ID NO: 26, a heavy chain CDR2 having an amino acid sequence of SEQ ID NO: 27, and a heavy chain CDR3 having an amino acid sequence of SEQ ID NO: 28, and a light chain variable region including a light chain CDR1 having an amino acid sequence of SEQ ID NO: 30, a light chain CDR2 having an amino acid sequence of SEQ ID NO: 31, and a light chain CDR3 having an amino acid sequence of SEQ ID NO: 32.

[Table 1]

| CD5 ScFv | Sequence |
|---|---|
| #1 (SEQ ID NO: 35) | MKYLLPTAAAGLLLLAAQPAMAEVQLVESGGGLVQPGGSLRLSCAASGFTFSDYGMHWVRQ<br>APGKGLEWVSAISGSGSSTHYADSVKGRFTISRDNSKNTLYLQMNSLRAEDTAVYYCAKGS<br>SSVYSEAMDLWGQGTLVTVSSGGGGSGGGGSGGGGSDIQMTQSPSSLSASVGDRVTITCRA<br>SQDISSYLNWYQQKPGKAPKLLIYATSNLQSGVPSRFSGSGSGTDFTLTISSLQPEDFATY<br>YCQQSYTYPWTFGQGTKVEIKASTGPGGQHHHHHHGAWSHPQFEK<br><br>CDR1-VH (SEQ ID NO: 2): DYGMH<br>CDR2-VH (SEQ ID NO: 3): AISGSGSSTHYADSVKG<br>CDR3-VH (SEQ ID NO: 4): GSSSVYSEAMDL<br>CDR1-VL (SEQ ID NO: 6): RASQDISSYLN<br>CDR2-VL (SEQ ID NO: 7): ATSNLQS<br>CDR3-VL (SEQ ID NO: 8): QQSYTYPWT |
| #4 (SEQ ID NO: 36) | MKYLLPTAAAGLLLLAAQPAMAEVQLVESGGGLVQPGGSLRLSCAASGFTFSDYAMSWVRQ<br>APGKGLEWVSAISQSGGSTYYADSVKGRFTISRDNSKNTLYLQMNSLRAEDTAVYYCAKDL<br>ASVYGAAFDVWGQGTLVTVSSGGGGSGGGGSGGGGSDIQMTQSPSSLSASVGDRVTITCRA<br>SQTIASYLNWYQQKPGKAPKLLIYAASTLQSGVPSRFSGSGSGTDFTLTISSLQPEDFATY<br>YCQQSYSFPWTFGQGTKVEIKASTGPGGQHHHHHHGAWSHPQFEK<br><br>CDR1-VH (SEQ ID NO: 10): DYAMS<br>CDR2-VH (SEQ ID NO: 11): AISQSGGSTYYADSVKG<br>CDR3-VH (SEQ ID NO: 12): DLASVYGAAFDV<br>CDR1-VL (SEQ ID NO: 14): RASQTIASYLN<br>CDR2-VL (SEQ ID NO: 15): AASTLQS<br>CDR3-VL (SEQ ID NO: 16): QQSYSFPWT |
| #11 (SEQ ID NO: 37) | MKYLLPTAAAGLLLLAAQPAMAEVQLVESGGGLVQPGGSLRLSCAASGFTFSSYAMSWVRQ<br>APGKGLEWVSAISSSGGSKYYADSVKGRFTISRDNSKNTLYLQMNSLRAEDTAVYYCAKRS<br>YAGEYFDHWGQGTLVTVSSGGGGSGGGGSGGGGSDIQMTQSPSSLSASVGDRVTITCRASQ<br>SISNWLNWYQQKPGKAPKLLIYDASSLQSGVPSRFSGSGSGTDFTLTISSLQPEDFATYYC<br>QQSYSFPWTFGQGTKVEIKASTGPGGQHHHHHHGAWSHPQFEK<br><br>CDR1-VH (SEQ ID NO: 18): SYAMS<br>CDR2-VH (SEQ ID NO: 19): AISSSGGSKYYADSVKG<br>CDR3-VH (SEQ ID NO: 20): RSYAGEYFDH<br>CDR1-VL (SEQ ID NO: 22): RASQSISNWLN<br>CDR2-VL (SEQ ID NO: 23): DASSLQS |

(continued)

| CD5 ScFv | Sequence |
|---|---|
| | CDR3-VL (SEQ ID NO: 24): QQSYSFPWT |
| #14 (SEQ ID NO: 38) | MKYLLPTAAAGLLLLAAQPAMAEVQLVESGGGLVQPGGSLRLSCAASGFTFSDYAMHWVRQ APGKGLEWVSAISSSGSYTYYADSVKGRFTISRDNSKNTLYLQMNSLRAEDTAVYYCAKGS YYGYYFDIWGQGTLVTVSSGGGGSGGGGSGGGGSDIQMTQSPSSLSASVGDRVTITCRASQ SISNDLNWYQQKPGKAPKLLIYDTSRLQSGVPSRFSGSGSGTDFTLTISSLQPEDFATYYC QQSYSFPWTFGQGTKVEIKASTGPGGQHHHHHHGAWSHPQFEK                CDR1-VH (SEQ ID NO: 26): DYAMH<br>CDR2-VH (SEQ ID NO: 27): AISSSGSYTYYADSVKG<br>CDR3-VH (SEQ ID NO: 28): GSYYGYYFDI<br>CDR1-VL (SEQ ID NO: 30): RASQSISNDLN<br>CDR2-VL (SEQ ID NO: 31): DTSRLQS<br>CDR3-VL (SEQ ID NO: 32): QQSYSFPWT |

**[1-2] Design of Chimeric Antigen Receptor**

A chimeric antigen receptor (CAR) was designed to have an extracellular domain including the four types of scFv designed as described above as antigen binding sites, and to be introduced into a natural killer cell using the CAR region of the sequence shown in Table 2 below. Specifically, the chimeric antigen receptor of the present invention was designed to have an intracellular signaling domain of the chimeric antigen receptor classified as the so-called thirdgeneration CAR by linking the extracellular domain including the four types of scFv as antigen binding sites and CD28 as a transmembrane domain via Myc and hinge domains, and adding and linking CD3-zeta as the intracellular signaling domain and CD28 DAP10 as a co-stimulatory molecule to the transmembrane domain (respectively referred to as "CD5#1-CAR," "CD5#4-CAR," "CD5#11-CAR," and "CD5#14-CAR," and collectively referred to as "CD5-CAR"). Then, the base sequence of the gene construct encoding the chimeric antigen receptor was inserted into the lentivirus vector (Clontech, 632155) (FIG. 1).

[Table 2]

| CAR region | Sequence |
|---|---|
| Signal peptide (SEQ ID NO: 43) | ACCATGGCCTTACCAGTGACCGCCTTGCTCCTGCCGCTGGCCTTACTGCTCCACG CCGCCAGGCCGGC |
| Myc (SEQ ID NO: 44) | GCGAACAAAAACTCATCTCAGAAGAGGATCTG |
| Hinge (SEQ ID NO: 45) | GGGGTCACCGTCTCTTCAGCGCTGAGCAACTCCATCATGTACTTCAGCCACTTCG TGCCGGTCTTCCTGCCAGCGAAGCCCACCACGACGCCAGCGCCGCGACCACCAAC ACCGGCGCCCACCATCGCGTCGCAGCCCCTGTCCCTGCGCCCAGAGGCATGCCGG CCAGCGGCGGGGGGCGCAGTGCACACGAGGGGGCTGGAT |
| Transmembrane (SEQ ID NO: 46) | GTGAAAGGGAAACACCTTTGTCCAAGTCCCCTATTTCCCGGACCTTCTAAGCCCT TTTGGGTGCTGGTGGTGGTTGGTGGAGTCCTGGCTTGCTATAGCTTGCTAGTAAC AGTGGCCTTTATTATTTTCTGGGTGAGGAGTAAGAGGAGCAGGCTCCTGCACAGT GACTACATGAACATGACTCCCCGCCGCCCCGGGCCCACCCGCAAGCATTACCAGC CCTATGCCCCACCACGCGACTTCGCAGCCTAT |
| DAP10 (SEQ ID NO: 47) | CTGTGCGCACGCCCACGCCGCAGCCCCGCCCAAGAAGATGGCAAAGTCTACATCA ACATGCCAGGCAGGGGC |

(continued)

| CAR region | Sequence |
|---|---|
| CD3ζ (SEQ ID NO: 48) | TAGAGTGAAGTTCAGCAGGAGCGCAGACGCCCCCGCGTACCAGCAGGGCCAGAAC CAGCTCTATAACGAGCTCAATCTAGGACGAAGAGAGGAGTACGATGTTTTGGACA AGAGACGTGGCCGGGACCCTGAGATGGGGGGAAAGCCGAGAAGGAAGAACCCTCA GGAAGGCCTGTACAATGAACTGCAGAAAGATAAGATGGCGGAGGCCTACAGTGAG ATTGGGATGAAAGGCGAGCGCCGGAGGGGCAAGGGGCACGATGGCCTTTACCAGG GTCTCAGTACAGCCACCAAGGACACCTACGACGCCCTTCACATGCAGGCCCTGCC CCCTCGCTAA |

## [1-3] Preparation of Natural Killer Cells Expressing CD5-CAR

The lentivirus vector prepared in Example 1-2 above was transformed into HEK293T cells together with viral packaging vectors (PMDLg/RRE, RSV/REV, and VSVG), and lentivirus expressing CD5-CAR was obtained therefrom. The lentivirus was concentrated by using a high-speed centrifuge, the concentrated lentivirus was infected to HEK293T cells, and then the amount of Myc epitope of CD5-CAR was confirmed with flow cytometry to calculate an infection unit. The number of natural killer cells and the amount of lentivirus were calculated so that the multiplicity of infection (MOI) became 30, and the lentivirus expressing CD5-CAR was infected to NK cells (NK92 cells) by spinoculation method (360 g, 90 min, and RT). The infected natural killer cells were cultured under conditions of 37 °C and 5% $CO_2$ for 5 hours, then transferred to a fresh culture medium, and after 3 days, the natural killer cells were treated with puromycin having a concentration of 3 ug/mL to select the natural killer cells that were properly infected, and the culture was continued.

As a control group, the uninfected natural killer cells were also treated with puromycin, and the culture was continued using a medium treated with the puromycin until the natural killer cells were all killed by the puromycin in the control group. When all of the natural killer cells of the control group were killed, the infected natural killer cells were selected, and the selected natural killer cells were cultured again in a medium without puromycin to be proliferated or expanded. For the proliferation or expansion of the selected natural killer cells, media using 12.5% fetal bovine serum containing Alpha-MEM, 12.5% equine serum, 0.2 mM inositol, 0.1 mM 2-mercaptoethanol, 0.02 mM folic acid and 200 U/mL recombinant IL-2 were used.

The expression of CD5-CAR was confirmed by treating the natural killer cells expressing CD5#1-CAR, CD5#4-CAR, CD5#11-CAR, and CD5#14-CAR, which were prepared and selected as described above (respectively referred to as "CD5#1-CAR-NK cells," "CD5#4-CAR-NK cells," "CD5#11-CAR-NK cells," and "CDS#14-CAR-NK cells," and collectively referred to as "CD5-CAR-NK cells") with anti-myc antibodies (CST; 9B11) specifically binding to the myc of CD5-CAR in the NK cells (4°C, and 30 minutes in the dark) with flow cytometry. In this case, the original natural killer cells not expressing CD5-CAR were used as a control group.

As a result, as shown in FIG. 2, it was confirmed that CD5-CAR was properly expressed in all four types of CD5-CAR-NK cells compared to the control group.

## [Example 2]

## Confirmation of Activity of Immune Cells Expressing CD5-CAR against Cancer Cells Expressing CD5

## [2-1] Confirmation of Activity of CD5-CAR-NK Cells against Cancer Cells Expressing CD5

For the cancer cell line MOLT4, which is known to express CD5, and the cancer cell line U937, which is known not to express CD5, the cytotoxicity (or cell-killing activity, cytolytic activity) of the four types of CD5-CAR-NK cells prepared in Examples 1-3 were confirmed with calcein AM analysis. Specifically, MOLT4 cells and U937 cells were treated and reacted with calcein-AM (life technologies; C1430) at a concentration of 5 ug/mL (37 °C, 5% $CO_2$, and 1 hour in the dark), and then the MOLT4 cells and U937 cells stained with calcein were treated with each of the four CD5-CAR-NK cells at a ratio of 2:1, 1:1, and 0.5:1 (natural killer cells:cancer cells) in 200 uL of RPMI1640 (10% FBS) (2 hours under 37°C and 5% $CO_2$), and then 100 uL of supernatant was taken to confirm the amount of calcein present in the supernatant, and the cytotoxicity according to each condition was calculated by the following method.

$$\text{Cytotoxicity (\%) = (Calcein release value-spontaneous value according to conditions) / (maximum value-spontaneous value) x 100}$$

As a control group, an empty lentivirus vector that did not include a gene construct encoding CD5-CAR of the present invention or a lentivirus vector that included a gene construct in which an extracellular domain was removed from CD5-CAR of the present invention was introduced into a natural killer cell in the same manner as in Example [1-3] above and the natural killer cells obtained through the process of selection with puromycin were used, and these cells are referred to as "PURO NK cells" and "dEcto-NK cells," respectively.

As a result, as shown in FIG. 3, it was confirmed that all four types of CD5-CAR-NK cells exhibited much higher cytotoxicity against the MOLT4 cells expressing CD5 than the control group (PURO NK cells or dEcto-NK cells), as well as such cytotoxicity was concentration-dependent on the treated CD5-CAR-NK cells. On the other hand, it was confirmed that both the control group and the four types of CD5-CAR-NK cells exhibited little cytotoxicity against U937 cells that did not express CD5.

In addition, two CD5-CAR-NK cells (CD5#11-CAR-NK cells and CD5#14-CAR-NK cells) that have been identified to have the best cytotoxicity among the above four types of CD5-CAR-NK cells were selected, and CCRF-CEM and Jurkat, which are different cancer cell lines known to express CD5, and Daudi, which is a different cancer cell line known not to express CD5, were treated with the respective two types of CD5-CAR-NK cells at a ratio of 2:1 (natural killer cells:cancer cells), thereby confirming again the cytotoxicity of CD5-CAR-NK cells by the same method as described above.

As a result, as shown in FIG. 4, it was confirmed that the two types of CD5-CAR-NK cells exhibited much higher cytotoxicity against both the CCRF-CEM cells and Jurkat cells expressing CD5 than the control group (PURO NK cells or dEcto-NK cells), whereas both the control group and the two types of CD5-CAR-NK cells exhibited little cytotoxicity against the Daudi cells which did not express CD5.

Furthermore, the secretion of cytokines and granules was confirmed with respect to the four types of CD5-CAR-NK cells. Specifically, the MOLT4 cells and U937 cells were treated with the Puro NK cells, the dEcto-NK cells, or each of the four CD5-CAR-NK cells prepared in Example 1-3 above at 1:1, followed by reaction in RPMI1640 (10% FBS) (12 hours under 37 °C and 5% $CO_2$ conditions), and then the supernatant was collected to identify IFN-γ, which is a cytokine present in the supernatant, through ELISA. In this case, the amount of cytokines secreted from Puro NK cells, dEcto-NK cells, and CD5-CAR-NK cells alone was used as a control group.

As a result, as shown in FIG. 5a, it was confirmed that the amount of INF-γ secreted was remarkably improved only in CD5-CAR-NK cells treated with the MOLT4 cells expressing CD5.

In addition, the MOLT4 cells and U937 cells were treated with the Puro NK cells, the dEcto-NK cells, or each of the four CD5-CAR-NK cells prepared in Example 1-3 above at 1:1, followed by reaction in RPMI1640 (10% FBS) (4 hours under 37°C and 5% $CO_2$ conditions), and treated with anti-CD56 antibody and stained to select natural killer cells, and the expression levels of CD107a in the Puro NK cells, the dEcto-NK cells, and the four types of CD5-CAR-NK cells were analyzed with flow cytometry.

As a result, as shown in FIGS. 5B to 5E, it was confirmed that the expression of CD107a was remarkably improved only in CD5-CAR-NK cells treated with the MOLT4 cells expressing CD5, as in the case of INF-γ.

**[2-2] Confirmation of Activity of CD5-CAR-NK Cells against Normal T Cells Expressing CD5**

In order to confirm what activity the CD5-CAR-NK cells of the present invention exhibit for normal T cells expressing CD5 rather than cancer cells expressing CD5, mononuclear cells (MNCs) was first separated from umbilical cord blood using the ficoll-paque® gradient method. Specifically, the umbilical cord blood was carefully placed onto ficoll so as not to be mixed, centrifuged at 2,000 rpm for 30 minutes at room temperature, and then a buffy coat (an enriched MNC fraction) containing white blood cells and platelets was separated. The fraction separated as described above was treated with the 1X ACK (Ammonium-Chloride-Potassium) dissolution buffer solution at 37 °C for 10 minutes to break the remaining red blood cells and separate only pure MNCs.

The separated MNCs were treated with the CD5#11-CAR-NK cells and the CD5#14-CAR-NK cells prepared in Example 1-3 above at a ratio of 1:1 (natural killer cells:MNCs), respectively, to confirm the cytotoxicity of the CD5-CAR-NK cells in the same manner as in Example [2-1] above.

As a result, as shown in FIG. 6, it was confirmed that both CD5#11-CAR-NK cells and CD5#14-CAR-NK cells exhibited decreased cytotoxicity against MNCs compared to MOLT4 cells, which are cancer cells expressing CD5.

**[2-3] Confirmation of Activity of CD5-CAR-NK Cells depending on Expression Level of CD5-CAR**

Each of the CD5#11-CAR-NK cells and the CD5#14-CAR-NK cells prepared in Example 1-3 above was separated into single cells in a 96-well plate using a high-speed flow cytometry sorter (BD FACS Aria Fusion). In addition, the cells proliferating at similar rates among them were repeatedly selected to proliferate in the order of a 48-well plate, a 24-well plate, a 6-well plate, and a 25-T flask.

Then, the expression level of CD5-CAR was checked for each of single cells of the separated CD5#11-CAR-NK cells and CD5#14-CAR-NK cells, and cell lines having different expression levels of CD5-CAR were constructed based on the results (FIG. 7a).

Then, the single cell lines of the CD5#11-CAR-NK cell and the CD5#14-CAR-NK cell constructed as described above were treated with the MOLT4 which is a cancer cell line expressing CD5 and the MNCs separated in Example [2-2] above at a ratio of 1:1 (natural killer cells:cancer cells or MNCs), respectively, and the cytotoxicity of the individual single cell lines of the CD5-CAR-NK cell was confirmed in the same manner as in Example [2-1] above.

As a result, as shown in FIG. 7b, it was confirmed that although the individual single cell lines of CD5-CAR-NK cells exhibited improved cytotoxicity against the MOLT4, which is a cancer cell expressing CD5, the cytotoxicity itself was not proportional to the expression level of CD5-CAR. In addition, for MNCs containing normal T cells, it was confirmed that the individual single cell lines of CD5#11-CAR-NK cells all exhibited decreased cytotoxicity, but the individual single cell lines of CD5#14-CAR-NK cells did not exhibit much decreased cytotoxicity.

From the above results, the individual single cell lines of CD5#11-CAR-NK cells exhibiting lower cytotoxicity than the normal T cells among the two CD5-CAR-NK cells were selected to exhibit high cytotoxicity against the MOLT4, which is a cancer cell expressing CD5, and low cytotoxicity against the MNCs including normal T cells, and as a result, the L4 single cell line was selected as a leading cell line as shown in FIG. 7c.

**[2-4] Confirmation of Activity of CD5-CAR-NK Cells Based on Primary Natural Killer Cells**

Further, it was confirmed whether the CD5-CAR-NK cells could exhibit excellent activity as confirmed in Examples [2-1] to [2-3] even when the CD5-CAR-NK cells of the present invention were prepared by using primary natural killer cells rather than NK92 cells.

To this end, as shown in FIG. 8, first, the mRNA structures of CD5#11-CAR and CD5#14-CAR having a structure for improving protein expression and mRNA safety by combining UTR of mRNA were designed and manufactured, and the base sequence of the mRNA structure was inserted into a lentivirus vector (Clontech, 632155). Then, mRNA of CD5-CAR having the structure shown in FIG. 8 was synthesized using the gene construct as a template by means of a mRNA synthesis kit (EZ™ High Yield In Vitro Transcription kit) .

The mRNA of CD5-CAR synthesized as described above was introduced into primary natural killer cells by means of electroporation, and after 7 hours, an anti-Myc antibody (CST; 9B11) specifically binding to Myc of CD5-CAR was treated (4 ° C, and 30 minutes in the dark), and the expression of Myc was confirmed with flow cytometry. As a result, as shown in FIG. 9, it was confirmed that CD5-CAR was properly expressed in the primary natural killer cells.

In addition, the MOLT4 and CCRF-CEM, which are cancer cell lines known to express CD5, and MNCs separated in Example [2-2] were treated with two types of CD5-CAR-NK cells prepared based on primary natural killer cells as described above at a ratio of 0.5:1 and 1:1 (primary natural killer cells:cancer cells), respectively, thereby confirming the cytotoxicity of primary natural killer cell-based CD5-CAR-NK cells in the same manner as in Example [2-1] above.

As a result, as shown in FIG. 10, it was confirmed that two types of primary natural killer cell-based CD5-CAR-NK cells both showed much higher cytotoxicity against the MOLT4 cells and CCRF-CEM cells which are cancer cells expressing CD5 than the control group (PURO NK cells or dEcto-NK cells), as well as such cytotoxicity was concentration-dependent on the treated CD5-CAR-NK cells. In contrast, it was confirmed that the MNCs containing normal T cells expressing CD5 exhibited further reduced cytotoxicity.

**[Example 3]**

**Confirmation of Ligand Regulating Activity of CD5-CAR-NK Cells of the Present Invention**

**[3-1] Ligand Screening for CD5-CAR-NK Cells of the Present Invention**

Although both normal T cells and T cell-based cancer cells express CD5, in order to find out why the CD5-CAR-NK cells of the present invention exhibit particularly high cytotoxicity against cancer cells expressing CD5, as confirmed in Example 2, the expression of the ligand involved in the activation of the MNCs separated in Example [2-2] above, which are normal cells, and MOLT4, CCRF-CEM, and Jurkat, which are the cancer cells expressing CD5. Each of the cells were treated with an antibody that specifically binds to each ligand, and the expression level of the corresponding ligand was analyzed with flow cytometry.

As a result, as shown in FIG. 11a, it was confirmed that among the various ligands, B7-H6 was expressed significantly high in all three types of the above-mentioned cancer cells, and HLA class1, which is an inhibitory ligand, was expressed high in normal cells.

In addition, the MNCs separated in Example [2-2] were treated with antibodies specifically binding to CD3, which is a marker for T cells, CD34, which is a marker for hematopoietic stem cells (HSCs), CD33, which is a marker for myeloid cells, and CD56, which is a marker for natural killer cells, respectively, and the expression level of B7-H6 for each type of white blood cells was measured with flow cytometry, and as a result, it was confirmed that B7-H6 was not expressed in most normal white blood cells, as shown in FIG. 11b.

[3-2] Confirmation of Effect of Expression of B7-H6 on Activity of CD5-CAR-NK Cells

It was confirmed what effect the expression of B7-H6, which is the ligand for activating the natural killer cells confirmed in Example [3-1] above had on the activity of the CD5-CAR-NK cells of the present invention.

To this end, siRNA having a sequence complementary to the gene of B7-H6 was introduced into MOLT4 cells and Jurkat cells, which are cancer cell lines expressing CD5, to thus knock-down the expression of B7-H6 temporarily. Then, the cancer cells in which the expression of B7-H6 was reduced as described above were treated with CD5#11-CAR-NK cells at a ratio of 1:1 (natural killer cells:cancer cells), respectively, thereby confirming the cytotoxicity of CD5#11-CAR-NK cells in the same manner as in Example [2-1] above.

As a result, as shown in FIG. 12, it was confirmed that the cytotoxicity of the CD5#11-CAR-NK cells was reduced in the cancer cells in which the expression of B7-H6 was reduced compared to the cancer cells in which the expression of B7-H6 was not reduced.

Further, the MOLT4 which is the cancer cell line expressing CD5 and the MNCs separated in Example [2-2] above were treated with CD5#11-CAR NK cells at a ratio of 1:1 (natural killer cells: cancer cells or MNCs), respectively, and the expression levels of B7-H6 and CD5 were measured in living MOLT4 cells and MNCs every 2 hours with flow cytometry.

As a result, as shown in FIG. 13, it was confirmed that the expression of B7-H6 and the expression of CD5 were reduced in living MOLT4 over time. From the above results, it may be seen that cells with high expression of B7-H6 were relatively early killed by CD5#11-CAR-NK cells.

**Discussion of [Example 2] and [Example 3]**

It has been confirmed that the CD5-CAR-NK cells of the present invention not only exhibit improved cytotoxicity in a concentration-dependent manner against the cancer cells expressing CD5, but also increase the secretion and degranulation of IFN-γ, and thus it may be seen that the CD5-CAR-NK cells of the present invention react specifically to the cancer cells expressing CD5.

CD5 is expressed in most T cell malignancies, but is also expressed in normal T cells, and it has been reported that all conventionally developed CD5-CAR have adverse effects on normal T cells. However, it was confirmed that the CD5-CAR-NK cells of the present invention exhibited further reduced cytotoxicity against the MNCs separated from umbilical cord blood, and in particular, it was confirmed that the CD5#11-CAR-NK cells exhibited lower cytotoxicity than the CD5#14-CAR-NK cells. The difference between the CD5#11-CAR-NK cells and the CD5#14-CAR-NK cells is considered due to the difference in the structure of the single chain variable fragment, and it is determined that the #11 ScFv including the heavy chain variable region including the heavy chain CDR1 having the amino acid sequence of SEQ ID NO: 18, the heavy chain CDR2 having the amino acid sequence of SEQ ID NO: 19, and the heavy chain CDR3 having the amino acid sequence of SEQ ID NO: 20, and the light chain variable region including the light chain CDR1 having the amino acid sequence of SEQ ID NO: 22, the light chain CDR2 having the amino acid sequence of SEQ ID NO: 23, and the light chain CDR3 having the amino acid sequence of SEQ ID NO: 24 have a more favorable effect on safety. In addition, it was confirmed that the cytotoxicity evaluation of individual cell lines established by separating and proliferating CD5-CAR-NK cells into single cells exhibited a similar pattern, and in particular, CD5#11-CAR-NK cells exhibited the cytotoxicity against cancer cells expressing CD5 about 10 times higher than the control group, whereas CD5#11-CAR-NK cells exhibited the cytotoxicity against normal cells expressing CD5 only about 2 times higher than the control group. From the above results, it is believed that the CD5#11-CAR-NK cells of the present invention have much higher safety than previously reported CD5-CARs. In addition, it was demonstrated that not only the CD5-CAR permanently expressed in NK92 cells but also the primary natural killer cell-based CD5-CAR-NK cells, which temporarily expressed CD5-CAR by introducing mRNA of CD5-CAR into the primary natural killer cells, have the cytotoxicity against cancer cells expressing CD5, thereby confirming the possibility of reducing the adverse effects in that even the normal cells expressing CD5 are reduced.

Meanwhile, in cancer cells expressing CD5 unlike normal cells, it was confirmed that B7-H6 was expressed particularly high among several ligands involved in the activation of natural killer cells, and from this, it was expected that the CD5-CAR-NK cells of the present invention react differently to cancer cells expressing CD5 and normal cells due to differences in the expression of B7-H6. Furthermore, it was confirmed that when the expression level of B7-H6 was adjusted in the cancer cells expressing CD5, the cytotoxicity of the CD5-CAR-NK cells of the present invention varied with the expression level of B7-H6, and from this, it may be seen that the B7-H6 acts as a major factor in the activity of the CD5-CAR-NK cells of the present invention and is expressed relatively high in the cancer cells expressing CD5, and thus the CD5-CAR-NK cells of the present invention respond to the cancer cells earlier than the normal cells.

In short, due to the difference in the activity of CD5-CAR depending on a difference in the CDR sequence of the antibody to CD5, the cytotoxicity against cancer cells expressing CD5 and normal cells may be totally differentiated, and it is believed that the single cell lines having different cytotoxicity depending on the expression level of CD5-CAR may be utilized to develop a new therapeutic agent for T cell blood cancer, the therapeutic agent exhibiting a high anticancer effect against cancer cells and low reactivity to normal cells.

As described above, the specific part of the present invention has been described in detail, and thus it will be apparent to those skilled in the art that the specific description is merely a preferred embodiment, and the scope of the present invention is not limited thereby. Accordingly, it will be considered that the substantial scope of the present invention is defined by the accompanying claims and equivalents thereof.

**Claims**

1. An antibody or an antigen-binding fragment thereof, specifically binding to CD5, comprising:

    a heavy chain variable region comprising i) a heavy chain CDR1 having any one amino acid sequence selected from the group consisting of SEQ ID NO: 2, SEQ ID NO: 10, SEQ ID NO: 18, and SEQ ID NO: 26, ii) a heavy chain CDR2 having any one amino acid sequence selected from the group consisting of SEQ ID NO: 3, SEQ ID NO: 11, SEQ ID NO: 19, and SEQ ID NO: 27, and iii) a heavy chain CDR3 having any one amino acid sequence selected from the group consisting of SEQ ID NO: 4, SEQ ID NO: 12, SEQ ID NO: 20, and SEQ ID NO: 28; and
    a light chain variable region comprising iv) a light chain CDR1 having any one amino acid sequence selected from the group consisting of SEQ ID NO: 6, SEQ ID NO: 14, SEQ ID NO: 22, and SEQ ID NO: 30, v) a light chain CDR2 having any one amino acid sequence selected from the group consisting of SEQ ID NO: 7, SEQ ID NO: 15, SEQ ID NO: 23, and SEQ ID NO: 31, and vi) a light chain CDR3 having any one amino acid sequence selected from the group consisting of SEQ ID NO: 8, SEQ ID NO: 16, SEQ ID NO: 24, and SEQ ID NO: 32.

2. The antibody or antigen-binding fragment thereof of claim 1, wherein the heavy chain variable region has any one amino acid sequence selected from the group consisting of SEQ ID NO: 5, SEQ ID NO: 13, SEQ ID NO: 21, and SEQ ID NO: 29.

3. The antibody or antigen-binding fragment thereof of claim 1, wherein the light chain variable region has any one amino acid selected from the group consisting of SEQ ID NO: 9, SEQ ID NO: 17, SEQ ID NO: 25, and SEQ ID NO: 33.

4. A chimeric antigen receptor (CAR) comprising an extracellular binding domain comprising an antigen-binding site specifically binding to CD5, a transmembrane domain, and an intracellular signaling domain, wherein the antigen-binding site specifically binding to the CD5 is a single chain variable fragment (scFv) of an anti-CD5 antibody comprising:

    a heavy chain variable region comprising i) a heavy chain CDR1 having any one amino acid sequence selected from the group consisting of SEQ ID NO: 2, SEQ ID NO: 10, SEQ ID NO: 18, and SEQ ID NO: 26, ii) a heavy chain CDR2 having any one amino acid sequence selected from the group consisting of SEQ ID NO: 3, SEQ ID NO: 11, SEQ ID NO: 19, and SEQ ID NO: 27, and iii) a heavy chain CDR3 having any one amino acid sequence selected from the group consisting of SEQ ID NO: 4, SEQ ID NO: 12, SEQ ID NO: 20, and SEQ ID NO: 28; and
    a light chain variable region comprising iv) a light chain CDR1 having any one amino acid sequence selected from the group consisting of SEQ ID NO: 6, SEQ ID NO: 14, SEQ ID NO: 22, and SEQ ID NO: 30, v) a light chain CDR2 having any one amino acid sequence selected from the group consisting of SEQ ID NO: 7, SEQ ID NO: 15, SEQ ID NO: 23, and SEQ ID NO: 31, and vi) a light chain CDR3 having any one amino acid sequence selected from the group consisting of SEQ ID NO: 8, SEQ ID NO: 16, SEQ ID NO: 24, and SEQ ID NO: 32.

5. The chimeric antigen receptor of claim 4, wherein the antigen-binding site specifically binding to the CD5 is a single chain variable fragment of an anti-CD5 antibody comprising a heavy chain variable region having any one amino acid sequence selected from the group consisting of SEQ ID NO: 5, SEQ ID NO: 13, SEQ ID NO: 21, and SEQ ID NO: 29.

6. The chimeric antigen receptor of claim 4, wherein the antigen-binding site specifically binding to the CD5 is a single chain variable fragment of an anti-CD5 antibody comprising a light chain variable region having any one amino acid sequence selected from the group consisting of SEQ ID NO: 9, SEQ ID NO: 17, SEQ ID NO: 25, and SEQ ID NO: 33.

7. A polynucleotide comprising a base sequence encoding the chimeric antigen receptor of any one of claims 4 to 6.

8. An expression vector comprising the polynucleotide of claim 7.

9. An immune cell expressing the chimeric antigen receptor of any one of claims 4 to 6 on the surface thereof.

10. The immune cell of claim 9, wherein the immune cell is at least one selected from a natural killer cell (NK cell), a T cell, a natural killer T cell (NKT cell), a macrophage, and a dendritic cell.

11. A pharmaceutical composition for preventing or treating cancer, the pharmaceutical composition comprising the immune cell of claim 9 as an active ingredient.

12. The pharmaceutical composition of claim 11, wherein the cancer is blood cancer comprising leukemia or lymphoma.

13. The pharmaceutical composition of claim 12, wherein the cancer is at least one selected from the group consisting of thymic carcinoma, non-Hodgkin lymphoma, diffuse large cell lymphoma, small lymphocytic lymphoma, T-cell neoplasm, peripheral T-cell lymphoma, mantle cell lymphoma, T-cell acute lymphoblastic leukemia, and chronic lymphocytic leukemia.

14. The pharmaceutical composition of claim 11, further comprising an anticancer chemotherapeutic agent.

FIG. 1

| ECTO DOMAIN | | | | ENDO DOMAIN | | |
|---|---|---|---|---|---|---|
| CD5 ScFv | myc | hinge | TM(CD28) | DAP10 | CD3 zeta |

FIG. 2

--- NK92

----- CD5-CAR NK92

FIG. 3

E:T ratio 2:1, 1:1, 0.5:1
incubation time: 2hr

MOLT-4

U937

FIG. 4

E:T ratio 2:1
incubation time: 2hr

CCRF-CEM

Specific lysis(%)

80.0
60.0
40.0
20.0
0.0

puro dEcto #11 #14

Jurkat

Specific lysis(%)

80.0
60.0
40.0
20.0
0.0

puro dEcto #11 #14

Daudi

Specific lysis(%)

100.0
80.0
60.0
40.0
20.0
0.0

puro dEcto #11 #14

FIG. 5a

E:T ratio 1:1
incubation time: 12hr

MOLT4

IFN-Y (pg/ml)

15

10

5

0

puro  dEcto  #1  #4  #11  #14

U937

IFN-Y (pg/ml)

10

8

6

4

2

0

puro  dEcto  #1  #4  #11  #14

puro

dEcto

**FIG. 5b**

only

U937

MOLT4

FIG. 5c

FIG. 5d

FIG. 5e

## FIG. 6

E:T ratio 1:1

incubation time: 2hr

FIG. 7a

CD5 CAR NK(#11)

CAR expression

CD5 CAR NK(#14)

CD5(#14)CAR expression

FIG. 7b

CD5 CAR NK(#11)

CD5 CAR NK(#14)

FIG. 7c

FIG. 8

## FIG. 9

FIG. 10

7 hours after transfection
E:T ratio 1:1
incubation time: 2hr

----- shock  ----- #11
·········· dEcto  ——— #14

FIG. 11a

– Ligand Expression associated with NK activation

FIG. 11b

- Analysis of B7-H6 level in Cord Blood

CD3 : T cell marker
CD34 : HSC marker
CD33 : myeloid cell marker
CD56 : NK cell marker

FIG. 12

FIG. 13

Time point: 0, 2, 4, 8h
E:T ratio 1:1
Condition: 3x10^5 + 3x10^5/1ml in 24well

Gating strategy

| INTERNATIONAL SEARCH REPORT | International application No. |
|---|---|
| | **PCT/KR2022/006009** |

**A. CLASSIFICATION OF SUBJECT MATTER**

**C07K 16/28**(2006.01)i; **C12N 5/0783**(2010.01)i; **A61K 35/17**(2014.01)i; **A61P 35/00**(2006.01)i

According to International Patent Classification (IPC) or to both national classification and IPC

**B. FIELDS SEARCHED**

Minimum documentation searched (classification system followed by classification symbols)

C07K 16/28(2006.01); A61K 31/195(2006.01); A61K 35/17(2014.01); A61K 38/17(2006.01); A61K 39/395(2006.01); A61K 45/06(2006.01); C12P 21/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: CD5, 항체(antibody), 키메라 항원 수용체(chimeric antigen receptor, CAR), 면역세포(immune cell), 암(cancer)

**C. DOCUMENTS CONSIDERED TO BE RELEVANT**

| Category* | Citation of document, with indication, where appropriate, of the relevant passages | Relevant to claim No. |
|---|---|---|
| A | WO 2020-023561 A1 (MAGENTA THERAPEUTICS, INC.) 30 January 2020 (2020-01-30) <br> See claims 1-14. | 1-14 |
| A | US 2020-0405811 A1 (BAYLOR COLLEGE OF MEDICINE) 31 December 2020 (2020-12-31) <br> See claims 1 and 19-32. | 1-14 |
| A | KR 10-2018-0002604 A (ICELL GENE THERAPEUTICS LLC et al.) 08 January 2018 (2018-01-08) <br> See claims 13-16. | 1-14 |
| A | WADA, M. et al. Characterization of an Anti-CD5 Directed CAR T-Cell against T-Cell Malignancies. Stem Cell Reviews and Reports. 01 February 2020, vol. 16, pp. 369-384. <br> See abstract; and figures 1-2. | 1-14 |
| A | US 2011-0250203 A1 (KLITGAARD, J. L. K. et al.) 13 October 2011 (2011-10-13) <br> See entire document. | 1-14 |

☐ Further documents are listed in the continuation of Box C.  ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **08 August 2022** | **08 August 2022** |

| Name and mailing address of the ISA/KR | Authorized officer |
|---|---|
| **Korean Intellectual Property Office** <br> **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

INTERNATIONAL SEARCH REPORT

International application No.

**PCT/KR2022/006009**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

   a. ☑ forming part of the international application as filed:

      ☑ in the form of an Annex C/ST.25 text file.

      ☐ on paper or in the form of an image file.

   b. ☐ furnished together with the international application under PCT Rule 13*ter*.1(a) for the purposes of international search only in the form of an Annex C/ST.25 text file.

   c. ☐ furnished subsequent to the international filing date for the purposes of international search only:

      ☐ in the form of an Annex C/ST.25 text file (Rule 13*ter*.1(a)).

      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (July 2019)

**INTERNATIONAL SEARCH REPORT**
Information on patent family members

International application No.

**PCT/KR2022/006009**

| Patent document cited in search report | | | Publication date (day/month/year) | Patent family member(s) | | | Publication date (day/month/year) |
|---|---|---|---|---|---|---|---|
| WO | 2020-023561 | A1 | 30 January 2020 | AU | 2019-311077 | A1 | 25 February 2021 |
| | | | | CA | 3107383 | A1 | 30 January 2020 |
| | | | | CN | 112739340 | A | 30 April 2021 |
| | | | | EP | 3826625 | A1 | 02 June 2021 |
| | | | | JP | 2021-532116 | A | 25 November 2021 |
| | | | | US | 2021-0355230 | A1 | 18 November 2021 |
| US | 2020-0405811 | A1 | 31 December 2020 | EP | 3286225 | A1 | 28 February 2018 |
| | | | | EP | 3286225 | A4 | 10 October 2018 |
| | | | | EP | 3286225 | B1 | 01 July 2020 |
| | | | | MA | 41962 | A | 28 February 2018 |
| | | | | US | 10786549 | B2 | 29 September 2020 |
| | | | | US | 2018-104308 | A1 | 19 April 2018 |
| | | | | WO | 2016-172606 | A1 | 27 October 2016 |
| KR | 10-2018-0002604 | A | 08 January 2018 | AU | 2016-225012 | A1 | 31 August 2017 |
| | | | | AU | 2016-225012 | B2 | 03 September 2020 |
| | | | | CA | 2977106 | A1 | 01 September 2016 |
| | | | | CN | 107249602 | A | 13 October 2017 |
| | | | | CN | 107249602 | B | 28 September 2021 |
| | | | | CN | 109414428 | A | 01 March 2019 |
| | | | | CN | 114230670 | A | 25 March 2022 |
| | | | | EP | 3261651 | A1 | 03 January 2018 |
| | | | | EP | 3261651 | B1 | 04 May 2022 |
| | | | | EP | 3419618 | A1 | 02 January 2019 |
| | | | | IL | 254141 | A | 31 October 2017 |
| | | | | JP | 2018-513692 | A | 31 May 2018 |
| | | | | JP | 2021-078514 | A | 27 May 2021 |
| | | | | SG | 11201706774 | A | 28 September 2017 |
| | | | | TW | 201706295 | A | 16 February 2017 |
| | | | | US | 10273280 | B2 | 30 April 2019 |
| | | | | US | 2018-0066034 | A1 | 08 March 2018 |
| | | | | US | 2019-0345217 | A1 | 14 November 2019 |
| | | | | WO | 2016-138491 | A1 | 01 September 2016 |
| | | | | WO | 2017-146767 | A1 | 31 August 2017 |
| US | 2011-0250203 | A1 | 13 October 2011 | AU | 2009-287164 | A1 | 04 March 2010 |
| | | | | BR | PI0917148 | A2 | 01 December 2015 |
| | | | | CA | 2735279 | A1 | 04 March 2010 |
| | | | | CN | 102137873 | A | 27 July 2011 |
| | | | | EP | 2328932 | A1 | 08 June 2011 |
| | | | | IL | 209975 | A | 28 February 2011 |
| | | | | JP | 2012-500815 | A | 12 January 2012 |
| | | | | KR | 10-2011-0050541 | A | 13 May 2011 |
| | | | | MX | 2011000970 | A | 15 March 2011 |
| | | | | NZ | 591153 | A | 21 December 2012 |
| | | | | RU | 2011111640 | A | 10 October 2012 |
| | | | | TW | 201011045 | A | 16 March 2010 |
| | | | | WO | 2010-022737 | A1 | 04 March 2010 |
| | | | | ZA | 201100300 | B | 25 January 2012 |

Form PCT/ISA/210 (patent family annex) (July 2019)

**REFERENCES CITED IN THE DESCRIPTION**

**Patent documents cited in the description**

- KR 20180002604
- KR 2122546

**Non-patent literature cited in the description**

- **YANOFSKY, C.** *J Bacteriol,* 1984, vol. 158, 1018-1024
- **HERSKOWITZ, I ; HAGEN, D.** *Ann Rev Genet,* 1980, vol. 14, 399-445
- *Appl Environ Microbiol,* 1998, vol. 64, 3932-3938
- *Mol Gen Genet,* 1996, vol. 250, 734-741